# EUROPEAN PATENT SPECIFICATION

(11) **EP 4 008 347 B1**
(45) Date of publication and mention of the grant of the patent: **30.04.2025**
(21) Application number: 20845660.8
(22) Date of filing: 31.07.2020
(51) Int. Cl.: A61K 39/395, A61K 48/00, A61P 35/00, A61P 37/04, A61P 43/00, C07K 14/725, C07K 16/30, C07K 19/00, C12N 5/10, C12N 15/12, C12N 15/13, C12N 15/62, A61K 38/17, A61K 35/17, C12P 21/08, A61K 39/00

(54) **GD2 BINDING MOLECULE**
GD2 BINDENDE MOLEKÜLE
MOLÉCULE SE LIANT AU GD2

(30) Priority: 01.08.2019 JP 2019142358
(43) Date of publication of application: 08.06.2022
(73) Proprietor: Mie University, Tsu-shi, Mie 514-8507 (JP)
(72) Inventor: SHIKU, Hiroshi, Tsu-shi, Mie 514-8507 (JP); AKAHORI, Yasushi, Tsu-shi, Mie 514-8507 (JP); MIWA, Hiroshi, Tsu-shi, Mie 514-8507 (JP); FUJIWARA, Hiroshi, Tsu-shi, Mie 514-8507 (JP); FURUKAWA, Koichi, Kasugai-shi, Aichi 487-8501 (JP); FURUKAWA, Keiko, Kasugai-shi, Aichi 487-8501 (JP); OHMI, Yuhsuke, Kasugai-shi, Aichi 487-8501 (JP)
(74) Representative: Gevers & Orès
(86) International application number: PCT/JP2020/029446
(87) International publication number: WO 2021/020564

(56) References cited:
- WO-A1-2012/033885
- WO-A1-2015/132604
- WO-A1-2016/134284
- WO-A1-2018/023025
- WO-A1-2018/237022
- WO-A1-2020/026284
- WO-A2-2018/183888
- JP-A- 2011 526 785
- JP-A- 2013 532 968
- JP-A- 2015 521 607
- FUKUDA M; HORIBE K; FURUKAWA K: "Enhancement of in vitro and in vivo anti-tumor activity of anti-GD2 monoclonal antibody 220-51 against human neuroblastoma by granulocyte-macrophage colony-stimulating factor and granulocyte colony- stimulating factor", INTERNATIONAL JOURNAL OF MOLECULAR MEDICINE, vol. 2, no. 4, 1 October 1998 (1998-10-01), pages 471 - 475, XP009526266, ISSN: 1107-3756, DOI: 10.3892/ijmm.2.4.471

## Description

### Technical Field

The present invention relates to a chimeric antigen receptor comprising a GD2-binding domain and the like.

### Background Art

Gangliosides are a family of glycolipids and are composed of a sugar chain portion and a lipid (ceramide: fatty acid + long-chain base). Gangliosides are synthesized by a series of enzymatic reactions and metabolized to end products. GD2 is synthesized from GD3 by GM2/GD2 synthase, and further synthesized to GD1b by GM1/GD1b/GA1 synthase.

In cancer cells, the expression of GD2 synthase is high, and the expression of GD1b synthase is low, resulting in high GD2 expression on the cell surface. It is known that GD2 expressed on cells is involved in cell adhesion and signal transduction by coexisting with adhesion molecules such as integrins and is involved in cancer growth and metastasis.

GD2 is known to be highly expressed in melanoma, neuroblastoma, glioblastoma, lung cancer, osteosarcoma, and leukemia. GD2 is expressed in nerve cells and glial cells in normal tissues, but its expression in these normal tissues is low.

### Citation List

### Patent Literature

PTL 1: WO2012/033885
PTL 2 : WO2018/237022 shows the generation of a GD2-CAR containing intracellular signalling domains from the co-stimulatory molecule CD28 and CD3 zeta. Said GD2-CAR was introduced into human T cells and expression of the GD2-CAR was confirmed. The GD2-CAR T cells are shown to have cytotoxic activity against a GD2-positive tumor cell line in an in vitro assay.

### Summary of Invention

### Technical Problem

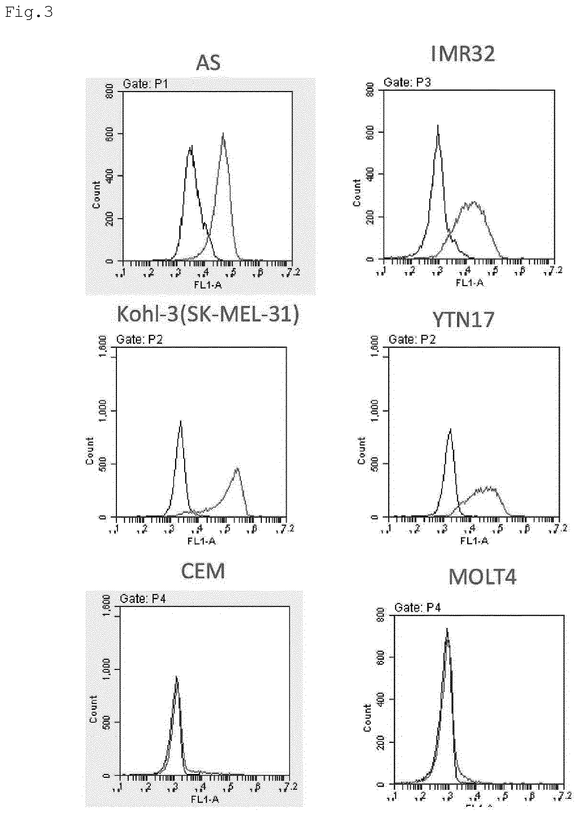

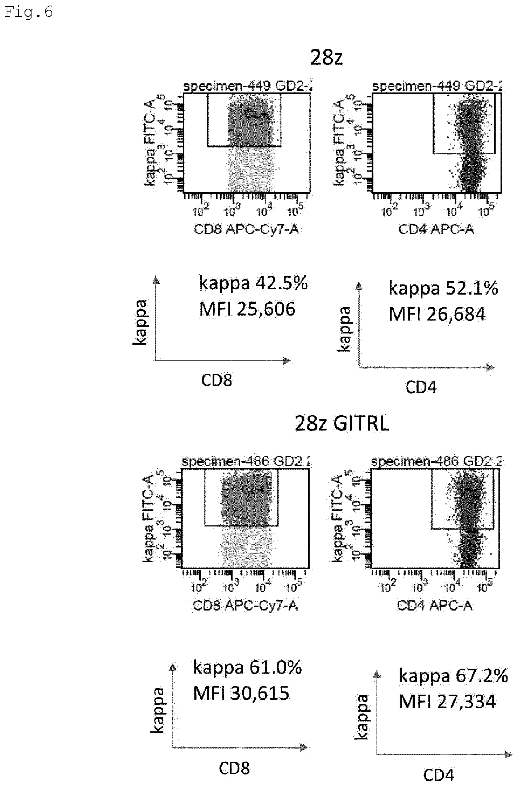

Because GD2 is considered to be a good molecule target, antibodies that recognize GD2 have been isolated and used in antibody treatment or CAR treatment (PTL 1). However, the treatments have limited effects so far, showing insufficient therapeutic efficacy. For example, as shown in Fig. 3 of PTL 1, cytotoxic action in vitro is weak, and the treatment on P1143 shows about 20% lysis (effector-to-target ratio: 5:1), which was the most potent effect, while showing only about a few percent for others. PTL 1 also discloses that melanoma was injected through IV to develop lung cancer, followed by effector infusion (1×10⁷) (Fig. 6), and 20% of mice were dead on day 100, showing that the therapeutic experiment did not achieve a complete cure.

An object of the present invention is to provide a cancer treatment or prevention technique that molecularly targets GD2.

### Solution to Problem

The present inventors conducted extensive research in view of the problem above and found that the problem can be solved by a chimeric antigen receptor comprising a GD2-binding domain comprising a heavy-chain variable region containing a heavy-chain CDR1 containing the amino acid sequence represented by SEQ ID NO: 1, a heavy-chain CDR2 containing the amino acid sequence represented by SEQ ID NO: 2, and a heavy-chain CDR3 containing the amino acid sequence represented by SEQ ID NO: 3, and a light-chain variable region containing a light-chain CDR1 containing the amino acid sequence represented by SEQ ID NO: 9, a light-chain CDR2 containing the amino acid sequence represented by SEQ ID NO: 10, and a light-chain CDR3 containing the amino acid sequence represented by SEQ ID NO: 11. The inventors conducted further research on the basis of this finding and completed the present invention. Specifically, the present invention includes the following subject matter.
The present invention relates to the object defined in claim 1. In one embodiment, the chimeric antigen receptor is characterized by its binding ability as defined in claim 2.
In another embodiment, the chimeric antigen receptor is characterized by its core domain as defined in claims 3 and 4.
In another embodiment, the chimeric antigen receptor a GITRL domain as defined in claim 5.
The present invention relates also to a polynucleotide as defined in claim 6.
The present invention relates also to a cell comprising the polynucleotide as defined in claim 7.
The present invention relates also to a chimeric antigen receptor T-cell or NK-cell as defined in claim 8.
The present invention relates to a pharmaceutical composition as defined in claims 9 and 10.

### Advantageous Effects of Invention

The present invention provides a technique of treating or preventing cancer that molecularly targets GD2. Specifically, the present invention treats or prevents cancer by using a chimeric antigen receptor that molecularly targets GD2.

### Brief Description of Drawings

Fig. 1 shows the ELISA results of Test Example 2. The vertical axis represents absorbance, and the horizontal axis represents the dilution factor. The legend shows immobilized antigens.

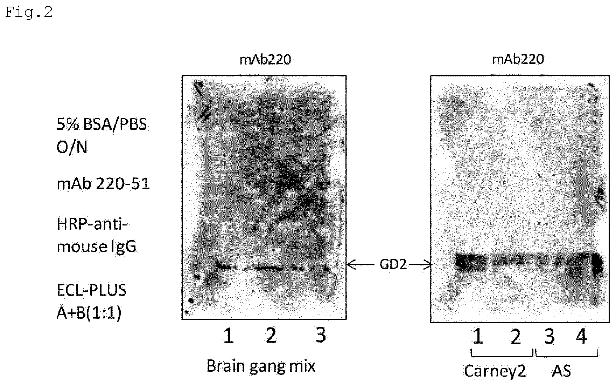

Fig. 2 shows the results of thin-layer chromatography and immunostaining in Test Example 3. Lanes 1, 2, and 3 in the left-hand photograph are lanes using 3 micrograms, 2 micrograms, and 1 microgram of a brain ganglioside mix, respectively. Lanes 1 and 2 in the right-hand photograph are lanes using 3 microliters and 1 microliter of a ganglioside extract of SK-MEL-23 (Carney2) cells (glycolipid extracted from 10 g of pellet and dissolved in 2 ml of C:M (1:1)). Lanes 3 and 4 in the right-hand photograph are lanes using 1 microliter and 3 microliters of an AS cell extract (glycolipid extracted from 1 g of pellet and dissolved in 0.5 ml of C:M (1:1)).
Fig. 3 shows the flow cytometry results of Test Example 4. The vertical axis represents cell count, and the horizontal axis represents fluorescence intensity. A black peak indicates a sample treated with 220-51 antibody, and a gray (red) peak indicates a sample not treated with 220-51 antibody. The cells used are shown above each histogram. AS, IMR32, Kohl-3 (SK-MEL-31), and YTN17 are GD2-positive cells, and CEM and MOLT4 are GD2-negative cells.
Fig. 4 shows the RT-CES analysis results of Test Example 5. The vertical axis represents the cell index (calculated from electrical resistance), and the horizontal axis represents the elapsed time from the start of measurement of cell adhesion.
Fig. 5 schematically shows the structures of four CARs (28z CAR, zG CAR, 28z GITRL CAR, and zG GITRL CAR).
Fig. 6 shows the results of Test Example 8 (expression in α/β cells when a 28z CAR or 28z GITRL CAR expression plasmid was introduced). The expression efficiency of anti-kappa CAR is shown as a percentage. The expression intensity of CAR-expressing cell fractions is shown by MFI.
Fig. 7 shows the results of Test Example 8 (expression in alpha/beta cells when a zG CAR or zG GITRL CAR expression plasmid was introduced). The expression efficiency of anti-kappa CAR is shown as a percentage. The expression intensity of CAR-expressing cell fractions is shown by MFI.
Fig. 8 shows the results of Test Example 8 (expression in alpha/beta cells when no CAR expression plasmid was introduced). The expression efficiency of anti-kappa CAR is shown as a percentage. The expression intensity of CAR-expressing cell fractions is shown by MFI.
Fig. 9 shows the results of Test Example 8 (expression in alpha/beta cells when a 28z GITRL CAR expression plasmid was introduced). The proportion of GITRL-expressing cells is shown as a percentage.
Fig. 10 shows the results of Test Example 8 (expression in alpha/beta cells when a zG GITRL CAR expression plasmid was introduced). The proportion of GITRL-expressing cells is shown as a percentage.
Fig. 11 shows the results of Test Example 8 (expression in alpha/beta cells when a CAR expression plasmid was introduced). The proportion of GITRL-expressing cells is shown as a percentage.
Fig. 12 shows the results of Test Example 8 (expression in gamma/delta cells when a 28z CAR or 28z GITRL CAR expression plasmid was introduced and when no CAR expression plasmid was introduced). The proportion of fractions that are kappa-positive and Vd2-positive is shown as a percentage.
Fig. 13 shows the results of Test Example 8 (expression in gamma/delta cells when a 28z GITRL CAR expression plasmid was introduced and when no CAR expression plasmid was introduced). The proportion of GITRL-expressing cells is shown as a percentage.
Fig. 14 shows target cell recognition of gamma/delta cells (the results of measurement of intracellular expression of IFNg and CD107a with a flow cytometer after co-culturing AS cells and CAR-T cells (in which a 28z CAR expression plasmid was introduced) for 4 hours (Test Example 9)). The proportions of IFNg-expressing cells and CD107a-expressing cells are shown as a percentage.
Fig. 15 shows target cell recognition of gamma/delta cells (the results of measurement of intracellular expression of IFNg and CD107a with a flow cytometer after co-culturing AS cells and CAR-T cells (in which a 28z GITRL CAR expression plasmid was introduced) for 4 hours (Test Example 9)). The proportions of IFNg-expressing cells and CD107a-expressing cells are shown as a percentage.
Fig. 16 shows target cell recognition of gamma/delta cells (the results of measurement of intracellular expression of IFNg and CD107a with a flow cytometer after co-culturing AS cells and PBMC for 4 hours (Test Example 9)). The proportions of IFNg-expressing cells and CD107a-expressing cells are shown as a percentage.
Fig. 17 shows the xCELLigence analysis results of Test Example 10 (alpha/beta cells). The vertical axis represents cytotoxic activity (%) measured by xCELLigence, and the horizontal axis represents the elapsed time from the addition of effector cells.
Fig. 18 shows the xCELLigence analysis results of Test Example 10 (gamma/delta cells). The vertical axis represents cytotoxic activity (%) measured by xCELLigence, and the horizontal axis represents the elapsed time from the addition of effector cells.
Fig. 19 shows the non-radioactive cytotoxicity test results of Test Example 10. The vertical axis represents the proportion of cytotoxic cells calculated based on the amount of luminescence. The ratio in the legend indicates the ratio of CAR-T cells to AS cells (number of CAR-T cells:number of AS cells).
Fig. 20 shows the results of Test Example 11. The vertical axis represents the cell index, which reflects the number of Kelly cells on an E-plate. The horizontal axis represents the elapsed time from the addition of the target cells.
Fig. 21 shows the results of Test Example 12. The vertical axis represents the cell index, which reflects the number of SK-N-SH cells on an E-plate. The horizontal axis represents the elapsed time from the addition of the target cells.
Fig. 22 shows the results of Test Example 13. The vertical axis represents the cell index, which reflects the number of Hs578T-Luc cells on an E-plate. The horizontal axis represents the elapsed time from the addition of the target cells.
Fig. 23 shows the results of Test Example 14. The vertical axis represents the cell index, which reflects the number of BT549-Luc cells on an E-plate. The horizontal axis represents the elapsed time from the addition of the target cells.
Fig. 24 shows the results of Test Example 15. The vertical axis represents the cell index, which reflects the number of Kelly cells on an E-plate. The horizontal axis represents the elapsed time from the addition of the target cells.
Fig. 25 shows the results of Test Example 16. The vertical axis represents the cell index, which reflects the number of D8 cells on an E-plate. The horizontal axis represents the elapsed time from the addition of the target cells.
Fig. 26 shows the results of Test Example 17. The vertical axis represents the cell index, which reflects the number of C2 cells on an E-plate. The horizontal axis represents the elapsed time from the addition of the target cells.
Fig. 27 shows the results of Test Example 18. The vertical axis represents the cell index, which reflects the number of NCI-N417 cells on an E-plate. The horizontal axis represents the elapsed time.

### Description of Embodiments

### 1. Definition

In the present specification, the terms "comprising," "containing," and "including" include the concepts of comprising, containing, consisting essentially of, and consisting of.

The "identity" of amino acid sequences refers to the degree to which two or more contrastable amino acid sequences match each other. Thus, the higher the degree of match between two amino acid sequences, the higher the identity or similarity of those sequences. The level of amino acid sequence identity is determined, for example, by using FASTA (a tool for sequence analysis) with default parameters. Alternatively, the level of amino acid sequence identity can be determined by using the BLAST algorithm by Karlin and Altschul (Karlin S, Altschul SF. Methods for assessing the statistical significance of molecular sequence features by using general scorings schemes, Proc Natl Acad Sci USA. 87: 2264-2268(1990), Karlin S, Altschul SF. Applications and statistics for multiple high-scoring segments in molecular sequences, Proc Natl Acad Sci USA.90: 5873-7(1993)). A program called "BLASTX," based on this BLAST algorithm, has been developed. The specific techniques of these analysis methods are known and can be found on the website of the National Center of Biotechnology Information (NCBI) (http://www.ncbi.nlm.nih.gov/). The "identity" of base sequences is also defined in the same manner as above.

In the present specification, "conservative substitution" means the substitution of an amino acid residue with an amino acid residue having a similar side chain. For example, the substitution between amino acid residues having a basic side chain such as lysine, arginine, or histidine is considered to be a conservative substitution. The following substitutions between other amino acid residues are also considered to be a conservative substitution: the substitution between amino acid residues having an acidic side chain such as aspartic acid or glutamic acid; the substitution between amino acid residues having an uncharged polar side chain such as glycine, asparagine, glutamine, serine, threonine, tyrosine, or cysteine; the substitution between amino acid residues having a nonpolar side chain such as alanine, valine, leucine, isoleucine, proline, phenylalanine, methionine, or tryptophan; the substitution between amino acid residues having a beta-branched side chain such as threonine, valine, or isoleucine; and the substitution between amino acid residues having an aromatic side chain such as tyrosine, phenylalanine, tryptophan, or histidine.

In the present specification, "CDR" is an abbreviation for complementarity determining region. CDR is a region in the variable regions of immunoglobulins and is deeply involved in the specific binding of an antibody to its antigen. The phrase "light-chain CDR" refers to a CDR present in the light-chain variable regions of immunoglobulins, and the phrase "heavy-chain CDR" refers to a CDR present in the heavy-chain variable regions of immunoglobulins.

In the present specification, the phrase "variable region" refers to a region containing CDR1 to CDR3 (simply "CDRs 1-3" below). The order in which these CDRs 1-3 are arranged is not limited; however, the variable region preferably refers to a region in which CDR1, CDR2, and CDR3 are arranged in this order in the direction from the N-terminus toward the C-terminus or in the reverse order either consecutively or via other amino acid sequences referred to as "framework regions" (FRs), which are described later. The phrase "heavy-chain variable region" refers to a region in which heavy-chain CDRs 1-3 are arranged, and the phrase "light-chain variable region" refers to a region in which light-chain CDRs 1-3 are arranged.

The regions other than CDRs 1-3 of each variable region are referred to as "framework regions" (FRs), as mentioned above. In particular, the region between the N-terminus and CDR1 of a variable region is defined as FR1, the region between CDR1 and CDR2 as FR2, the region between CDR2 and CDR3 as FR3, and the region between CDR3 and the C-terminus of a variable region as FR4.

### 2. GD2-binding Molecule

In an embodiment, the present invention relates to a chimeric antigen receptor comprising a GD2-binding domain comprising a heavy-chain variable region containing a heavy-chain CDR1 containing the amino acid sequence represented by SEQ ID NO: 1, a heavy-chain CDR2 containing the amino acid sequence represented by SEQ ID NO: 2, and a heavy-chain CDR3 containing the amino acid sequence represented by SEQ ID NO: 3; and a light-chain variable region containing a light-chain CDR1 containing the amino acid sequence represented by SEQ ID NO: 9, a light-chain CDR2 containing the amino acid sequence represented by SEQ ID NO: 10, and a light-chain CDR3 containing the amino acid sequence represented by SEQ ID NO: 11 (in the present specification, "the GD2-binding molecule of the present invention"). The chimeric antigen receptor comprising a GD2-binding domain of the present invention is described below.

The chimeric antigen receptor comprising a GD2-binding domain of the present invention can be any chimeric antigen receptor comprising a GD2-binding domain as long as the chimeric antigen receptor comprising a GD2-binding domain contains a heavy-chain variable region containing a heavy-chain CDR1 containing the amino acid sequence represented by SEQ ID NO: 1, a heavy-chain CDR2 containing the amino acid sequence represented by SEQ ID NO: 2, and a heavy-chain CDR3 containing the amino acid sequence represented by SEQ ID NO: 3, and a light-chain variable region containing a light-chain CDR1 containing the amino acid sequence represented by SEQ ID NO: 9, a light-chain CDR2 containing the amino acid sequence represented by SEQ ID NO: 10, and a light-chain CDR3 containing the amino acid sequence represented by SEQ ID NO: 11, and as long as the GD2-binding molecule is capable of binding to GD2.

The chimeric antigen receptor comprising a GD2-binding domain of the present invention may be a molecule formed of a single type of polypeptide or a molecule formed of a complex of two or more types of polypeptides. The chimeric antigen receptor comprising a GD2-binding domain of the present invention may also be a molecule formed of a polypeptide or of a complex of polypeptides, or a molecule formed of a polypeptide or complex of polypeptides to which another substance (e.g., a fluorescent substance, a radioactive substance, or an inorganic particle) is linked.

The binding capability to GD2 can be measured in accordance with a known method, for example, by ELISA (specifically, for example, by the method of Test Example 2). The binding capability of the GD2-binding molecule of the present invention to GD2 is, for example, at least 20%, at least 50%, at least 70%, at least 80%, at least 90%, at least 95%, or at least 99% of the binding capability of 220-51 antibody to GD2 in the Examples described later, which are taken as 100%.

The GD2-binding molecule of the present invention preferably contains both the heavy-chain variable region and the light-chain variable region.

The heavy-chain variable region is preferably a heavy-chain variable region containing the amino acid sequence represented by SEQ ID NO: 4, or an amino acid sequence having at least 90% (preferably at least 95%, preferably at least 98%, preferably at least 99%) identity with the amino acid sequence represented by SEQ ID NO: 4. The light-chain variable region is preferably a light-chain variable region containing the amino acid sequence represented by SEQ ID NO: 12, or an amino acid sequence having at least 90% (preferably at least 95%, preferably at least 98%, preferably at least 99%) identity with the amino acid sequence represented by SEQ ID NO: 12. If the amino acid sequence of SEQ ID NO: 4 or 12 is mutated, the mutation is preferably a substitution of an amino acid, and more preferably a conservative substitution of an amino acid.

The chimeric antigen receptor comprising a GD2-binding domain of the present invention can specifically recognize ganglioside GD2. From this viewpoint, the binding capability of the GD2-binding molecule of the present invention to other antigens, which are at least one member selected from the group consisting of ganglioside GD1a, ganglioside GD1b, ganglioside GD3, ganglioside GM1, ganglioside GM3, ganglioside GT1b, and lactosylceramide (preferably, two members or more, three members or more, four members or more, five members or more, six members or more, or seven members (all)), is preferably 1/2 or less (preferably, 1/5 or less, 1/10 or less, 1/20 or less, 1/100 or less, 1/500 or less, 1/2000 or less, or 1/10000 or less) of the binding capability of the GD2-binding molecule of the present invention to ganglioside GD2.

The chimeric antigen receptor comprising a GD2-binding domain of the present invention may be chemically modified. The polypeptide that constitutes the GD2-binding molecule of the present invention may have a carboxyl group (-COOH), carboxylate (-COO-), amide (-CONH₂), or ester (-COOR) at the C-terminus. "R" in the ester is, for example, a C₁₋₆ alkyl group such as methyl, ethyl, n-propyl, isopropyl, or n-butyl; a C₃₋₈ cycloalkyl group such as cyclopentyl or cyclohexyl; a C₆₋₁₂ aryl group such as phenyl or α-naphthyl; a phenyl-C₁₋₂ alkyl group such as benzyl or phenethyl; a C₇₋₁₄ aralkyl group such as an α-naphthyl-C₁₋₂ alkyl group such as α-naphthyl methyl; or a pivaloyloxymethyl group. The polypeptide that constitutes the GD2-binding molecule of the present invention may have an amidated or esterified carboxyl group (or carboxylate), which is not the carboxyl group at the C-terminus. The ester in this case may be, for example, the esters of the C-terminus described above. The polypeptide that constitutes the GD2-binding molecule of the present invention further includes polypeptides having the amino group of the N-terminal amino acid residue protected by a protective group (e.g., a C₁₋₆ acyl group including a C₁₋₆ alkanoyl such as a formyl group and an acetyl group), polypeptides having the N-terminal glutamine residue pyroglutamated that can be formed due to cleavage in vivo; and polypeptides having a substituent (e.g., -OH, -SH, an amino group, an imidazole group, an indole group, and a guanidino group) on a side change of an amino acid in the molecule protected by an appropriate protective group (e.g., a C₁₋₆ acyl group including a C₁₋₆ alkanoyl group such as a formyl group and an acetyl group).

The chimeric antigen receptor comprising a GD2-binding domain of the present invention may have a protein or peptide (e.g., a known protein tag or signal sequence) added. Examples of protein tags include biotin, a His tag, a FLAG tag, a Halo tag, a MBP tag, a HA tag, a Myc tag, a V5 tag, a PA tag, and a fluorescent protein tag.

The chimeric antigen receptor comprising a GD2-binding domain of the present invention may be a pharmaceutically acceptable salt formed with an acid or base. The salt can be any pharmaceutically acceptable salt, and can be either an acid salt or a basic salt. Examples of acid salts include inorganic acid salts, such as hydrochloride, hydrobromide, sulfate, nitrate, and phosphate; organic acid salts, such as acetate, propionate, tartarate, fumarate, maleate, malate, citrate, methanesulfonate, and para-toluenesulfonate; and amino acid salts, such as aspartate, and glutamate. Examples of basic salts include alkali metal salts such as sodium salts and potassium salts; and alkaline-earth metal salts, such as calcium salts and magnesium salts.

The chimeric antigen receptor comprising a GD2-binding domain of the present invention may be in the form of a solvate. The solvent can be any pharmaceutically acceptable solvent, and may be, for example, water, ethanol, glycerol, or acetic acid.

### 2-1. Antibody - object that is not part of the invention

According to embodiments that are not part of the invention, the GD2-binding molecule is an antibody (in the present specification, the GD2-binding molecule being an antibody may be referred to as "the antibody").

The antibody is a monoclonal antibody.

The antibody can be of any molecular weight. The lower limit is, for example, 20,000, preferably 50,000, preferably 100,000, and more preferably 120,000. The upper limit is, for example, 1,000,000, preferably 500,000, and more preferably 200,000.

The antibody may be of any structure. The antibody may contain constant regions, or no constant region. If the antibody contains constant regions, the antibody may contain all of the constant regions of the heavy chain (CH1, CH2, and CH3) and the constant regions of the light chain (CL), or any one or a combination of two or more constant regions of these constant regions.

Specific examples of the structure of the antibody include immunoglobulins, Fab, F(ab')₂, minibody, scFv-Fc, Fv, scFv, diabody, triabody, and tetrabody. Of these, an immunoglobulin is preferable from the standpoint of the effect.

An immunoglobulin has a structure formed of a combination of two structures each of which is composed of a single heavy chain that contains a heavy-chain variable region and a heavy-chain constant region and a single light chain that contains a light-chain variable region and a light-chain constant region.

"Fab" contains a fragment of a heavy chain containing the heavy-chain variable region and CH1 in the heavy-chain constant region and a light chain containing the light-chain variable region and the light-chain constant region (CL), with the heavy-chain variable region and the light-chain variable region being aggregated by non-covalent intermolecular interaction described above, or bound to each other through a disulfide bond. In Fab, CH1 and CL may be linked through a disulfide bond between the thiol groups of the cysteine residues present in CH1 and CL.

"F(ab')₂" contains two pairs of Fabs, with CH1 of one Fab linked with CH1 of the other Fab through a disulfide bond between the thiol groups of their cysteine residues.

"Minibody" refers to the structure in which two fragments each containing CH3 bound to a heavy-chain variable region constituting scFV, described below, are aggregated between CH3 and CH3 by non-covalent intermolecular interaction.

"scFv-Fc" refers to the structure in which two antibody fragments each containing scFv, CH2, and CH3 are aggregated between CH3 and CH3 by non-covalent intermolecular interaction, as with the minibody, and the fragments are linked through a disulfide bond between thiol groups of the cysteine residues contained in each CH3.

"Fv" is considered to be the smallest structural unit of an antibody with the heavy-chain variable region and the light-chain variable region being aggregated by non-covalent intermolecular interaction. In Fv, the thiol group of the cysteine residue present in the heavy-chain variable region may be linked to the thiol group of the cysteine residue present in the light-chain variable region through a disulfide bond.

"scFv" has the structure in which the C-terminus of the heavy-chain variable region and the N-terminus of the light-chain variable region are bound through a linker, or the N-terminus of the heavy-chain variable region and the C-terminus of the light-chain variable region are bound through a linker, and is also referred to as a "single-chain antibody."

The "diabody," "triabody," and "tetrabody" respectively refer to a dimer, a trimer, and a tetramer formed by scFv described above and are each aggregated and structurally stabilized, for example, by non-covalent intermolecular interaction of the variable regions, as with Fv.

If the antibody is an immunoglobulin, its class is not particularly limited. The classes include, for example, IgA, IgD, IgE, IgG, and IgM, as well as subclasses of these classes. The class of the antibody is, for example, IgG or IgM, preferably IgG, and more preferably IgG1.

The origin of the antibody is not particularly limited. The antibody may be, for example, a human-derived antibody, a mouse-derived antibody, a rat-derived antibody, a rabbit-derived antibody, a monkey-derived antibody, or a chimpanzee-derived antibody. The antibody may be a chimeric antibody (e.g., an antibody formed by replacing the amino acid sequence of the constant region of an antibody derived from a non-human organism (e.g., a mouse) with the amino acid sequence of the constant region of a human-derived antibody), a humanized antibody, or a fully humanized antibody.

The antibody can be produced, for example, by a method including culturing a host transformed with a polynucleotide encoding the antibody, and collecting the fraction containing the antibody.

The polynucleotide encoding the antibody can be any polynucleotide that expressibly contains the sequence of the antibody, and may contain other sequences in addition to the coding sequence of the antibody. Other sequences include a secretory-signal-peptide-coding sequence, a promoter sequence, an enhancer sequence, a repressor sequence, an insulator sequence, an origin of replication, and a drug-resistant-gene-coding sequence that are located adjacent to the coding sequence of the antibody. The polynucleotide encoding the antibody may also be a linear polynucleotide or a cyclic polynucleotide (e.g., a vector).

Specific examples of polynucleotides include (I) polynucleotides containing a base sequence encoding at least one member selected from the group consisting of the heavy chain, the heavy-chain variable region, the heavy-chain CDR1, the heavy-chain CDR2, and the heavy-chain CDR3 of the antibody, (II) polynucleotides containing a base sequence encoding at least one member selected from the group consisting of the light chain, the light-chain variable region, the light-chain CDR1, the light-chain CDR2, and the light-chain CDR3 of the antibody, (III) polynucleotides containing a base sequence encoding at least one member selected from the group consisting of the heavy chain, the heavy-chain variable region, the heavy-chain CDR1, the heavy-chain CDR2, and the heavy-chain CDR3 of the antibody, and polynucleotides containing a base sequence encoding at least one member selected from the group consisting of the light chain, the light-chain variable region, the light-chain CDR1, the light-chain CDR2, and the light-chain CDR3 of the antibody.

The host can be any organism, and is, for example, insect cells, eukaryotic cells, or mammal cells. Of these, mammal cells such as HEK cells, CHO cells, NS0 cells, SP2/O cells, or P3U1 cells are preferable from the standpoint of more efficiently expressing the antibody. The methods for transformation, culture, and collection are not particularly limited, and any method known in the field of antibody production can be used. After being collected, the antibody may optionally be purified. Purification can be performed by a method known in the field of antibody production, such as chromatography or dialysis.

### 2-2. Chimeric Antigen Receptor

In a preferable embodiment, the chimeric antigen receptor comprising a GD2-binding domain the present invention is a chimeric antigen receptor. (In the present specification, the chimeric antigen receptor comprising a GD2-binding domain of the present invention may be referred to as "the chimeric antigen receptor of the present invention.")

The chimeric antigen receptor (CAR) is typically a chimeric protein that has its single-chain antibody (scFv) composed of a light chain (VL) bound in tandem to a heavy chain (VH) of the variable region of a monoclonal antibody at a position closer to the N-terminus as a domain responsible for its binding capability to an antigen and its T-cell receptor (TCR) ζ chain at a position closer to the C-terminus. T cells expressing CAR are referred to as "CAR-T cells."

The domain responsible for the binding capability to an antigen (GD2) (GD2-binding domain) in the chimeric antigen receptor of the present invention is not particularly limited as long as the domain contains a heavy-chain variable region containing a heavy-chain CDR1 containing the amino acid sequence represented by SEQ ID NO: 1, a heavy-chain CDR2 containing the amino acid sequence represented by SEQ ID NO: 2, and a heavy-chain CDR3 containing the amino acid sequence represented by SEQ ID NO: 3, and a light-chain variable region containing a light-chain CDR1 containing the amino acid sequence represented by SEQ ID NO: 9, a light-chain CDR2 containing the amino acid sequence represented by SEQ ID NO: 10, and a light-chain CDR3 containing the amino acid sequence represented by SEQ ID NO: 11.

The GD2-binding domain preferably has the structure of scFv. The linker that links the heavy-chain variable region with the light-chain variable region can be any linker that maintains functionality of the chimeric antigen receptor. The linker is preferably a GS linker (typically, a linker having a repeated sequence containing GGGGS (SEQ ID NO: 41) as a structural unit). The number of amino acid residues of the linker is, for example, 5 to 30, preferably 10 to 20, and more preferably 15.

The chimeric antigen receptor of the present invention typically contains a core domain containing a scFv domain having a heavy-chain variable region and a light-chain variable region, a transmembrane domain, and the intracellular domain of TCR. In the core domain, the scFv domain, the transmembrane domain, and the intracellular domain of TCR are arranged in this order from the N-terminus directly or via other domains.

The transmembrane domain can be of any type that does not interfere with the functionality of the chimeric antigen receptor. For example, CD28, CD3zeta, CD4, or CD8alpha, which are expressed in cells such as T cells, can be used. These transmembrane domains may be mutated as long as the functionality of the chimeric antigen receptor is not interfered with.

The intracellular domain of TCR can be, for example, an intracellular domain derived from CD3, which is also called a "TCRζ chain." CD3 may be mutated as long as the functionality of the chimeric antigen receptor is not interfered with. Mutation of CD3 is preferably made such that CD3 contains ITAM (immunoreceptor tyrosine-based activation motif).

The chimeric antigen receptor of the present invention preferably has a spacer sequence between the scFv domain and the transmembrane domain. The spacer sequence can be of any length and can be formed of any amino acid residues as long as the functionality of the chimeric antigen receptor is not interfered with. For example, the spacer sequence can be designed so as to have about 10 to 200 amino acid residues. The spacer sequence for use is preferably the sequence of the constant region of the light chain.

The core domain in the chimeric antigen receptor of the present invention preferably further contains the intracellular domain of a co-stimulator. The intracellular domain of a co-stimulator can be of any intracellular domain derived from a co-stimulator of cells such as T cells. For example, at least one member selected from the group consisting of OX40, 4-1BB, GITR, CD27, CD278, CD28 and the like can be suitably selected and used. The intracellular domain of these co-stimulators may be mutated as long as the functionality of the chimeric antigen receptor is not interfered with. The position of the intracellular domain of a co-stimulator is not particularly limited as long as the intracellular domain is at a position closer to the C-terminus of the transmembrane domain; the intracellular domain may be at a position closer to the N-terminus or the C-terminus of the intracellular domain of TCR.

The chimeric antigen receptor of the present invention preferably contains a ligand domain such as a GITRL domain, a 4-1BBL domain, or an ICOSL domain at a position closer to the C-terminus of the core domain via a self-cleaving peptide domain. This can increase the expression efficiency of the chimeric antigen receptor or the cytotoxic activity of CAR-T cells containing the chimeric antigen receptor.

In the present specification, the phrase "self-cleaving peptide" refers to a peptide sequence with cleavage activity occurring between two amino acid residues in the peptide sequence. Examples of self-cleaving peptides include 2A peptides and 2A-like peptides. For example, in 2A peptides or 2A-like peptides, cleavage occurs between the glycine residue and the proline residue of these peptides. This occurs because of the "ribosomal skipping mechanism," in which a normal peptide linkage between the glycine residue and the proline residue does not form during translation, and this does not affect the translation downstream. The ribosomal skipping mechanism is known in the art and is used in the expression of multiple proteins encoded by a single molecular messenger RNA (mRNA). The self-cleaving peptide for use in the present invention can be obtained from 2A peptides of viruses or 2A-like peptides that have equivalent functionality. For example, the self-cleaving peptide can be selected from the group consisting of 2A peptides derived from foot-and-mouth disease virus (FMDV) (F2A), 2A peptides derived from equine rhinitis A virus (ERAV) (E2A), 2A peptides derived from porcine teschovirus (PTV-1) (P2A), and 2A peptides derived from *Thosea asigna* virus (TaV) (T2A). The self-cleaving peptide domain may be mutated as long as the activity of the self-cleaving peptide domain is not greatly impaired.

The GITRL domain is not particularly limited. The GITRL domain is, for example, preferably a domain having the amino acid sequence represented by SEQ ID NO: 40, or an amino acid sequence having at least 90% identity (preferably at least 95%, preferably at least 98%, and preferably at least 99%) with the amino acid sequence represented by SEQ ID NO: 40. If the GITRL domain is an amino acid sequence having a mutation in the amino acid sequence represented by SEQ ID NO: 40, the mutation is preferably a substitution of an amino acid, and more preferably a conservative substitution of an amino acid.

The techniques for producing a chimeric antigen receptor and a CAR-T cell that expresses the chimeric antigen receptor are known. Chimeric antigen receptors and CAR-T cells can be produced in accordance with a known method or an equivalent method.

### 3. Polynucleotide

In an embodiment, the present invention relates to a polynucleotide encoding the chimeric antigen receptor comprising a GD2-binding domain of the present invention (which may be referred to as "the polynucleotide of the present invention" in the present specification). The polynucleotide of the present invention is described below.

The polynucleotide of the present invention may contain other sequences in addition to the coding sequence of the chimeric antigen receptor comprising a GD2-binding domain of the present invention. Preferably, the polynucleotide of the present invention expressibly contains the sequence of the chimeric antigen receptor comprising a GD2-binding domain of the present invention. Other sequences include promoter sequences, enhancer sequences, repressor sequences, insulator sequences, origins of replication, reporter protein (e.g., fluorescent proteins) coding sequences, and drug-resistant-gene-coding sequences. The polynucleotide of the present invention may be a linear polynucleotide or a cyclic polynucleotide (e.g., a vector). The vector can be a plasmid vector or a virus vector (e.g., an adenovirus or retrovirus). The vector can also be, for example, a vector for cloning or for expression. The vector for expression includes vectors for prokaryotic cells, such as *Escherichia coli,* or actinomycetes, and vectors for eukaryotic cells, such as yeast cells, insect cells, or mammal cells.

The polynucleotide of the present invention includes not only DNA and RNA but also known chemically modified DNA or RNA as described below. To prevent the degradation by hydrolases such as nucleases, the phosphate residue (phosphate) of each nucleotide can be substituted with, for example, a chemically modified phosphate residue such as phosphorothioate (PS), methylphosphonate, or phosphorodithionate. The hydroxyl group at position 2 of the ribose of each ribonucleotide may also be substituted with -OR (R represents, for example, CH3(2'-O-Me), CH₂CH₂OCH₃ (2'-O-MOE), CH₂CH₂NHC(NH)NH₂, CH₂CONHCH₃, or CH₂CH₂CN). Additionally, the base moiety (pyrimidine, purine) may be chemically modified, by, for example, introduction of a methyl group or a cationic functional group into positon 5 of the pyrimidine base, or substitution of the carbonyl group at position 2 with thiocarbonyl. Additionally, the polynucleotide of the present invention also includes, but is not limited to, those formed by modifying the phosphate moiety or the hydroxyl portion, for example, by biotin, an amino group, a lower alkyl amine group, or an acetyl group. The term "polynucleotide" includes not only natural nucleic acids but also BNA (bridged nucleic acid), LNA (locked nucleic acid), and PNA (peptide nucleic acid).

### 4. Cell

In an embodiment, the present invention relates to a cell comprising the polynucleotide of the present invention (which may be referred to as "the cell of the present invention" in the present specification). The cell of the present invention is described below.

The cells from which the cell of the present invention is derived are not particularly limited.

When the cell of the present invention comprises a polynucleotide encoding the chimeric antigen receptor of the present invention, the cell is preferably a T cell. The T cell is preferably a cell expressing the chimeric antigen receptor of the present invention. In a more specific embodiment of the T cell of the present invention, the chimeric antigen receptor of the present invention is expressed on the cell membrane, and preferably expressed in such a state that the chimeric antigen receptor comprising a GD2-binding domain is exposed outside the cell membrane.

A T cell or the like expressing the chimeric antigen receptor recognizes GD2 in the GD2-binding domain, and then intracellularly transfers a recognition signal to activate a signal that induces cytotoxic activity. In conjunction with this, the cell mounts attacks against other cells or tissues expressing GD2, or exerts cytotoxic activity.

When a cell exhibiting such a function is a CTL, this cell is called a "chimeric antigen receptor T-cell" ("CAR-T cell"). Cells that have potential to exhibit cytotoxic activity, such as NK cells, can also exert cytotoxic activity when the GD2-binding domain binds to GD2, as with the chimeric antigen receptor T-cell. Thus, a host cell comprising the polynucleotide encoding the chimeric antigen receptor (in particular, a host cell having cytotoxic activity) is useful as an active ingredient of pharmaceutical compositions.

Such CAR-T cells or the like are useful for treatment or prevention of cancer or the like because they specifically recognize cancer tissue (tumor tissue). The type of cancer is not particularly limited, and includes solid cancer and blood cancer. Examples of solid cancer include lung cancer, colorectal cancer, ovarian cancer, breast cancer, brain tumor, stomach cancer, liver cancer, tongue cancer, thyroid cancer, kidney cancer, prostate cancer, uterine cancer, osteosarcoma, chondrosarcoma, rhabdomyosarcoma, melanoma, neuroblastoma, bladder cancer, and the like.

The cell of the present invention can be obtained by introducing the polynucleotide of the present invention into cells. If necessary, the cell containing the polynucleotide of the present invention may be concentrated, or may be concentrated using a specific marker (CD antigen, such as CD8) as an indicator.

### 5. Pharmaceutical Composition

In an embodiment, the present invention relates to a pharmaceutical composition comprising the chimeric antigen receptor T-cell or chimeric antigen receptor NK-cell containing the polynucleotide encoding the chimeric antigen receptor of the present invention, (which may be referred to as "the pharmaceutical composition of the present invention" in the present specification). The pharmaceutical composition of the present invention is described below.

The content of the cell in the pharmaceutical composition can be appropriately set in consideration of the type of target disease (e.g., solid cancer), desired therapeutic effects, administration method, treatment period, patient's age, patient's body weight, etc. The content of the cell in the pharmaceutical composition may be, for example, about 1 cell/mL to 10⁴ cells/mL.

The administration form of the pharmaceutical composition is not particularly limited as long as the desired effects are obtained. The pharmaceutical composition can be administered to mammals, including humans, by any of the following administration routes: oral administration and parenteral administration (e.g., intravenous injection, intramuscular injection, subcutaneous administration, rectal administration, dermal administration, and local administration). Since the active ingredient is a cell, the administration form is preferably parenteral administration, and more preferably intravenous injection. The dosage forms for oral administration and parenteral administration, and their production methods are well known to a person skilled in the art. The pharmaceutical composition can be produced according to a usual method by, for example, mixing the cell of the present invention with a pharmaceutically acceptable carrier etc.

Examples of dosage forms for parenteral administration include injection preparations (e.g., intravenous drip infusion, intravenous injection, intramuscular injection, subcutaneous injection, and endodermic injection), external preparations (e.g., ointments, cataplasms, and lotions), suppositories, inhalants, eye drops, ophthalmic ointments, nasal drops, ear drops, liposome agents, and the like. For example, an injection preparation can be prepared by dissolving or suspending cells in distilled water for injection, and optionally adding a solubilizer, a buffer, a pH adjuster, an isotonizing agent, a soothing agent, a preservative, a stabilizer, etc. The pharmaceutical composition can also be used as a freeze-dried preparation prepared before use.

The pharmaceutical composition may further comprise other drugs effective for the treatment or prevention of diseases. The pharmaceutical composition can also contain components such as sterilants, antiphlogistics, cell activators, vitamins, and amino acids, if necessary.

As the carrier used for formulating the pharmaceutical composition, excipients, binders, disintegrators, lubricants, coloring agents, and flavoring agents that are generally used in this technical field can be used; and stabilizers, emulsifiers, absorption enhancers, surfactants, pH adjusters, antiseptics, antioxidants, extenders, moisturizers, surface activators, dispersants, buffers, preservatives, solubilizers, soothing agents, and the like can also optionally be used.

The type of disease treated or prevented using the pharmaceutical composition is not particularly limited as long as the treatment or prevention can be achieved. Examples of specific target diseases include tumors. Preferable examples of tumors include GD2-positive tumors. The type of tumor is not particularly limited, and includes solid cancer and blood cancer. Examples of solid cancer include lung cancer (in particular, small-cell lung cancer), colorectal cancer, ovarian cancer, breast cancer, brain tumor, stomach cancer, liver cancer, tongue cancer, thyroid cancer, kidney cancer, prostate cancer, uterine cancer, osteosarcoma, chondrosarcoma, rhabdomyosarcoma, melanoma, neuroblastoma, bladder cancer, and the like.

The administration target (test subject) of the pharmaceutical composition is, for example, an animal having a disease described above or an animal with a potential to develop such a disease. A "potential to develop such a disease" can be determined by a known diagnostic method. The animal is, for example, a mammal, and preferably a human.

The dose of the pharmaceutical composition can be determined by a clinical physician, taking into consideration various factors, such as administration route, the type of disease, the degree of symptoms, patient's age, sex, and body weight, severity of disease, pharmacological findings such as pharmacokinetics and toxicological characteristics, use or non-use of drug delivery system, and whether the composition is administered as part of a combinational drug with other medicinal agents. When the active ingredient is the cell, the dose can be about 10⁴ cells/kg (body weight) to 10⁹ cells/kg (body weight). The administration schedule of the pharmaceutical composition can also be determined taking into consideration the same factors as those for the dose. For example, the composition can be administered once a day to once a month in the daily dose described above.

### Examples

The present invention is described in detail below with reference to Examples. However, the present invention is not limited to these Examples.

### Materials and Experimental Methods

Unless otherwise specified, the following materials and methods were used in the Test Examples.

### (1) Cell

Carney and AS were obtained from Dr. Old. IMR32, CEM, Kokl-3, and MOLT4 were obtained from Dr. Old/Ueda. YTN17 was provided by Dr. Yodoi, and subline N1 of SK-MEL-28 cells was provided by Dr. Lloyd. NCI-417, ACC-LC-171, ACC-LC-96, and ACC-LC-17 were provided by Dr. Takashi Takahashi. C-2 cells D-18 were prepared by introducing GD3 synthase into ACC-LC-17. GD2-expressing cells S1 and S6 were prepared by introducing, into subline N1 (GD3, not expressing GD3) of SK-MEL-28 cells, pCDNA3.1neo into which GD3 synthase and GM2/GD2 synthase cDNAs were incorporated. V4 and V9 are those into which empty vector pCDNA3.1neo was introduced.

### (2) Antibody

A rabbit anti-human kappa antibody (159) was purchased from MBL. An Alexa 488-labeled anti-rabbit IgG antibody (A11034) was purchased from Invitrogen. A PE-labeled anti-GITRL antibody (FAB6941P) was purchased from BioLegend. A PE-labeled anti-human 4-1BB antibody (311504) was purchased from BioLegend. A PE-labeled anti-human ICOSL antibody (309404) was purchased from BioLegend. An APC-labeled anti-human CD4 antibody (clone RPA-T4) was purchased from Invitrogen. A PE-labeled anti-human CD4 antibody (555347) was purchased from BD. An APC/Cy7-labeled anti-human CD8 antibody (clone HT8a) was purchased from BioLegend. A FITC-labeled anti-human Vd2 antibody (clone B6, 331418) was purchased from BioLegend. A V450-labeled anti-human IFNg antibody (clone 45.83, 48-7319-42) was purchased from BD Pharmingen. A PE/Cy7-labeled anti-human TNFa antibody (clone Mab11, 12-7349-82) was purchased from eBioscience. An APC-labeled anti-human CD107a antibody (560664) was purchased from BD Pharmingen.

### (3) Construction of CAR Expression Plasmid, Preparation of Retrovirus, Map, and Sequence

CD1928 and CD1928z GITRL prepared by Eurofins were subjected to enzymatic treatment with restriction enzymes NotI and XhoI, and recombined to pMS3 to prepare plasmid vectors. Luciferase NGFR expression vectors were prepared by treating these two prepared by Eurofins' custom synthesis with NotI and ClaI, and ClaI and XhoI, respectively, and recombining them into pMS3. These were introduced into Plat-A using FuGENE to prepare retroviruses. The method was performed according to the manufacturer's instructions.

### (4) Culture of PBMC and Retroviral Gene Transfer

After 2 micrograms of OKT3 and 10 micrograms of RetroNectin were immobilized on a 12-well plate, peripheral blood mononuclear cells adjusted with Ficoll were cultured in GT-T551 supplemented with 0.6% human plasma and IL-2 at a final concentration of 600 u/ml, collected on day 4, infected with retroviruses immobilized at 42°C for 2 hours at 2000×g, and cultured.

Gamma/delta cells were made according to the method of Tanaka et al. Gamma/delta cells (obtained by culturing peripheral blood mononuclear cells in YM-AB containing a novel bisphosphonate preparation (PTA), adding 25 ng/ml of IL-7 and 25 ng/ml of IL-15, and collecting them on day 4) were infected and cultured in the same medium.

### (5) Confirmation of CAR and GITRL Expression

For CAR expression, an anti-kappa antibody was reacted at 10 micrograms/ml, followed by washing; Alexa 488-labeled anti-rabbit IgG (Invitrogen) was reacted at 5 micrograms/ml, followed by washing; staining with an APC/Cy7-labeled anti-human CD8 antibody (BD) and an APC-labeled anti-human CD4 antibody (BioLegend) was performed; and measurement was performed with a FACSCanto. For GITRL expression, PE-labeled anti-human GITRL (BioLegend) was diluted 100-fold and reacted, and measurement was performed with a FACSCanto. Intracellular staining of GITRL with BD Cytofix/Cytoperm and BD Perm/Wash was performed using a PE-labeled anti-human GITRL antibody (BioLegend). The method was performed according to the manufacturer's instructions.

### (6) Intracellular Staining

After CAR-transduced PBMCs and target cells were mixed, the cells were reacted with an APC-labeled anti-human CD107a antibody, and cultured in a CO₂ incubator for 1 hour. Thereafter, GolgiStop was allowed to act, and culture was performed in a CO₂ incubator for 4 hours, followed by washing. Staining with an anti-human kappa antibody and an Alexa 488-labeled anti-rabbit IgG antibody was performed, and then staining with an APC/Cy7-labeled anti-CD8 antibody and a PE-labeled anti-human CD4 antibody was performed. After treatment with BD Cytofix/Cytoperm and BD Perm/Wash, staining was performed with V450-labeled anti-human IFNg and PE/Cy7-labeled anti-human TNFa.

### (7) xCELLigence Measurement

1.5×10⁴ target cells AS suspended in 100 microliters of RPMI 1640 10% FCS were placed and allowed to stand in a CO₂ incubator for 24 hours. Thereafter, 1.5×10⁴ effector cells suspended in 100 microliters of RPMI 1640 10% FCS were placed, and the subsequent changes in current were recorded.

### (8) Non-radioactive Cytotoxicity Measurement

The experiment was performed according to the manufacturer's instructions. Specifically, first, 4×10⁵ target AS cells were suspended in 400 microliters of 10% FCS/PRMI 1640, and 1 microliter of a BM-HT solution was added thereto, followed by culturing in a CO₂ incubator for 15 minutes. After washing, 5×10³ cells were prepared, and 50×10³, 15×10³, and 5×10³ CAR-T cells were added thereto, and the cells were co-cultured for 2 hours and then centrifuged to collect 25 microliters of a supernatant. 250 microliters of an EU solution was added thereto, followed by mixing. Thereafter, luminescence was measured with a TriStar2 SLB942 Multimode Reader (Berthold Technologies).

### Test Example 1: Isolation of Monoclonal Antibody

A Balb/c x C57BL/6 F1 mouse was immunized with three subcutaneous inoculations of IMR32 cells, and the collected spleen cells were fused with NS-1 cells, followed by culturing in RPMI 1640 medium containing 10% FCS and HAT, thereby obtaining monoclonal antibodies. The obtained antibodies were screened by flow cytometry recognition for IMR32 cells. Subclones of the obtained clone 220 were further obtained, and 220-51 was obtained.

### Test Example 2: Antigen Specificity Analysis 1

The antigen specificity of the 220-51 antibody was analyzed by ELISA. Gangliosides GD1a, GD1b, GD2, GD3, GM1, GM3, and GT1b, and lactosylceramide (50 ng each) were immobilized with methanol. Each serially diluted ascites antibody was reacted, and an HRP-labeled anti-mouse IgG antibody (Southern Biotech) was reacted. Color was developed using OPD, and the absorbance was measured.

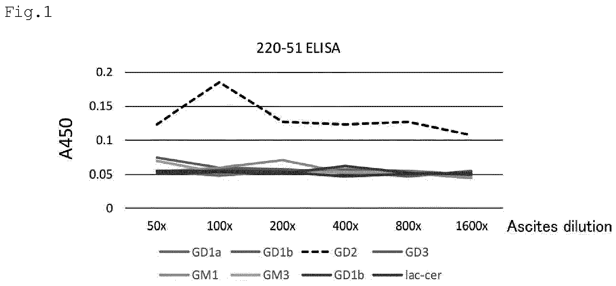

Fig. 1 shows the results. The 220-51 antibody recognized only GD2 and did not recognize any of the other gangliosides.

### Test Example 3. Antigen Specificity Analysis 2

The antigen specificity of the 220-51 antibody was analyzed by thin-layer chromatography. A mixture of a bovine-derived ganglioside and GM3, and gangliosides derived from cancer cells SK-MEL-23 (Carney2) and AS were subjected to thin-layer chromatography and transferred onto a PVDF membrane with a heat blotter (ATTO TLC Thermal Blotter AC5970, Atto, Tokyo), and the 220-51 antibody was then reacted. Thereafter, HRP-conjugated anti-mouse IgG (whole) (Cell Signaling), which is an HRP-labeled anti-mouse secondary antibody, was reacted, followed by light emission with a Western Lightning^{™} Plus ECL (PerkinElmer Inc., Waltham, MA).

Fig. 2 shows the results. The 220-51 antibody recognized only GD2 and did not recognize any of the other gangliosides.

### Test Example 4: Recognition of GD2-expressing Cell

1×10⁵ cells were treated with the 100-fold diluted antibody in 0.5% BSA/PBS at room temperature for 30 minutes, washed, treated with a FITC-labeled anti-mouse IgG antibody (Cappel), washed with PBS, and measured with a FACS Caliver or Accuri C6.

Fig. 3 shows the results. The 220-51 antibody recognized GD2+ AS, IMR32, Kohl-3 (SK-MEL-31), and YTN17, but did not recognize GD2- CEM or MOLT4.

### Test Example 5: Cell Adhesion Inhibition

GD2+ melanoma S1 and S6 cells, and GD2- V4 and V9 cells (number of cells: 1×10⁴) were seeded on a plate on which collagen was immobilized, and adhesion was observed with an RT-CES when the 220-51 antibody was diluted 50-fold and reacted at 0.5 hours and 3 hours (S1-T: addition of antibody to S1).

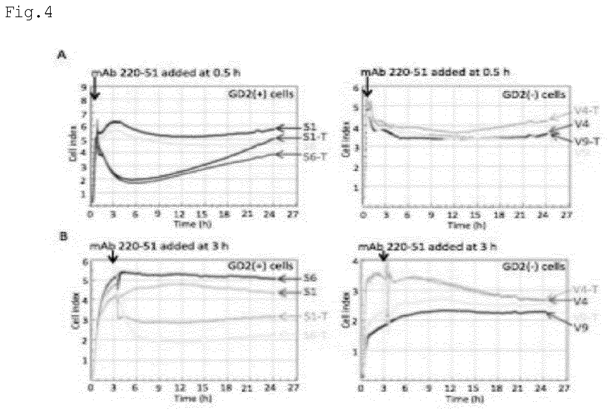

Fig. 4 shows the results. The 220-51 antibody inhibited the adhesion of GD2+ S1 and S6 cells, but did not inhibit the adhesion of GD2- V4 or V9 cells.

### Test Example 6: Sequence Analysis

The amino acid sequence of the 220-51 antibody and the base sequence encoding the antibody were analyzed. The analysis results are shown below. The sequences of the CDRs were deduced by IMGIT.

### Heavy Chain

Heavy-chain CDR1 amino acid sequence: GFSLPSYG (SEQ ID NO: 1)
Heavy-chain CDR2 amino acid sequence: IWAGGITN (SEQ ID NO: 2)
Heavy-chain CDR3 amino acid sequence: ARGGSDYDGFAY (SEQ ID NO: 3)
Heavy-chain variable region amino acid sequence: 
EVQLVESGPGLVAPSQSLSITCTVSGFSLPSYGVHWVRQPPGKGLEWLGVIWAGGITNYNSALMS
RLTISKDNSKSQVFLKMNSLQTDDTAIYYCARGGSDYDGFAYWGQGTLVTVS (SEQ ID NO:
4)
Heavy-chain CDR1 base sequence: GGG TTT TCA TTA CCC AGC TAT GGT (SEQ ID NO: 5)
Heavy-chain CDR2 base sequence: ATC TGG GCT GGT GGA ATC ACA AAT (SEQ ID NO: 6)
Heavy-chain CDR3 base sequence: GCC AGA GGC GGC TCT GAT TAC GAC GGC TTT GCT TAC (SEQ ID NO: 7)
Heavy-chain variable region base sequence:

GAGGTGCAGCTGGTGGAGTCTGGACCTGGCCTGGTGGCGCCCTCACAGAGCCTGTCCATCACTTG
CACTGTCTCTGGGTTTTCATTACCCAGCTATGGTGTTCACTGGGTTCGCCAGCCTCCAGGAAAGG
GTCTGGAGTGGCTGGGAGTAATCTGGGCTGGTGGAATCACAAATTATAACTCGGCTCTCATGTCC
AGACTGACCATCAGCAAAGACAACTCCAAGAGCCAAGTTTTCTTAAAAATGAACAGTCTTCAAAC
TGATGACACAGCCATATACTACTGTGCCAGAGGCGGCTCTGATTACGACGGCTTTGCTTACTGGG
GCCAAGGGACTCTGGTCACTGTCTCTGCATCA (SEQ ID NO: 8)

### Light Chain

Light-chain CDR1 amino acid sequence: QSLLSSRTRKNY (SEQ ID NO: 9)
Light-chain CDR2 amino acid sequence: WAS (SEQ ID NO: 10)
Light-chain CDR3 amino acid sequence: KQSYNLRT (SEQ ID NO: 11)
Light-chain variable region amino acid sequence: 
DIVMTQSPSSLAVSAGEKVTMNCRSSQSLLSSRTRKNYLAWYQQKPGQSPKLLIYWASIRESGVP
DRFTGSGSGTDFTLTISSVQAEDLAVYYCKQSYNLRTFGGGTKLEIK (SEQ ID NO: 12)
Light-chain CDR1 base sequence: CAG AGT CTC CTC AGC AGT AGA ACC CGA AAG AAC TAC (SEQ ID NO: 13)
Light-chain CDR2 base sequence: TGG GCA TCT (SEQ ID NO: 14) Light-chain CDR3 base sequence: AAG CAA TCT TAT AAT CTT CGG ACG (SEQ ID NO: 15)
Light-chain variable region base sequence:

GACATTGTGATGACACAGTCTCCATCCTCCCTGGCTGTGTCAGCAGGAGAGAAGGTCACTATGAA
CTGCAGATCCAGTCAGAGTCTCCTCAGCAGTAGAACCCGAAAGAACTACTTGGCTTGGTACCAGC
AGAAACCAGGGCAGTCTCCTAAACTGCTGATCTACTGGGCATCTATTAGGGAATCTGGGGTCCCT
GATCGCTTCACAGGCAGTGGATCTGGGACAGATTTCACTCTCACCATCAGCAGTGTGCAGGCTGA
AGACCTGGCAGTTTATTACTGCAAGCAATCTTATAATCTTCGGACGTTCGGTGGAGGCACCAAGC
TGGAAATCAAA (SEQ ID NO: 16)

### Test Example 7: Construction of CAR Expression Plasmid

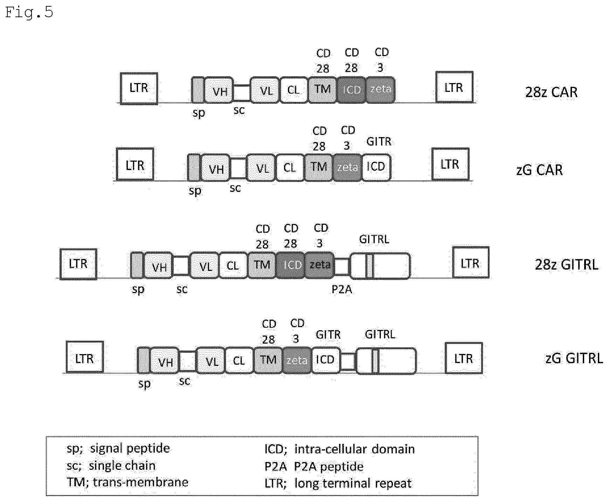

Four CARs (28z CAR, zG CAR, 28z GITRL CAR, and zG GITRL CAR) were designed using the amino acid sequence of the 220-51 antibody (Fig. 5 schematically shows the structures). Expression plasmids for these CARs were prepared. Specifically, the expression plasmids were prepared as follows.

For 28z CAR and zG CAR, artificial genes of the following two sequences were created by Eurofins, excised with NotI and XhoI, and inserted into pMS3 to obtain expression plasmids.

### Artificial Gene Base Sequence for Preparing 28z CAR

NotI site kozak sequence: GCGGCCGCCACC (SEQ ID NO: 17)-mVH leader:
ATGAACTTTGGGCTCAGATTGATTTTCCTTGTCCTTACTTTAAAAGGTGTGAAGTGT (SEQ ID NO: 18)-
mVH: 
GAGGTGCAGCTGGTGGAGTCTGGACCTGGCCTGGTGGCGCCCTCACAGAGCCTGTCCATCACTTG
CACTGTCTCTGGGTTTTCATTACCCAGCTATGGTGTTCACTGGGTTCGCCAGCCTCCAGGAAAGG
GTCTGGAGTGGCTGGGAGTAATCTGGGCTGGTGGAATCACAAATTATAACTCGGCTCTCATGTCC
AGACTGACCATCAGCAAAGACAACTCCAAGAGCCAAGTTTTCTTAAAAATGAACAGTCTTCAAAC
TGATGACACAGCCATATACTACTGTGCCAGAGGCGGCTCTGATTACGACGGCTTTGCTTACTGGG
GCCAAGGGACTCTGGTCACTGTCTCTGCATCA (SEQ ID NO: 19)-
single chain: GGAGGTGGAGGTTCTGGTGGAGGAGGTTCAGGTGGAGGTGGATCA(SEQ ID NO: 20)-
mVkappa: 
GACATTGTGATGACACAGTCTCCATCCTCCCTGGCTGTGTCAGCAGGAGAGAAGGTCACTATGAA
CTGCAGATCCAGTCAGAGTCTCCTCAGCAGTAGAACCCGAAAGAACTACTTGGCTTGGTACCAGC
AGAAACCAGGGCAGTCTCCTAAACTGCTGATCTACTGGGCATCTATTAGGGAATCTGGGGTCCCT
GATCGCTTCACAGGCAGTGGATCTGGGACAGATTTCACTCTCACCATCAGCAGTGTGCAGGCTGA
AGACCTGGCAGTTTATTACTGCAAGCAATCT TAT AAT CTT CGG ACG TTC GGT GGA
GGC ACC AAG CTG GAA ATC AAA (SEQ ID NO: 21)-
hCkappa: 
CGAACTGTGGCTGCACCATCTGTCTTCATCTTCCCGCCATCTGATGAGCAGTTGAAATCTGGAAC
TGCCTCTGTTGTGTGCCTGCTGAATAACTTCTATCCCAGAGAGGCCAAAGTACAGTGGAAGGTGG
ATAACGCCCTCCAATCGGGTAACTCCCAGGAGAGTGTCACAGAGCAGGACAGCAAGGACAGCACC
TACAGCCTCAGCAGCACCCTGACGCTGAGCAAAGCGGACTACGAGAAACACAAACTCTACGCCTG
CGAAGTCACCCATCAGGGCCTGAGCTCGCCCGTCACAAAGAGCTTCAACAGGGGAGAGTGTGGCG
CGCCA (SEQ ID NO: 22)-
hCD28 transmembrane: 
ACTAGATTTTGGGTGCTGGTGGTGGTTGGTGGAGTCCTGGCTTGCTATAGCTTGCTAGTAACAGT
GGCCTTTATTATTTTCTGGGTGAGG (SEQ ID NO: 23)-
hCD28 intracellular domain: 
AGTAAGAGGAGCAGGCTCCTGCACAGTGACTACATGAACATGACTCCCCGCCGCCCCGGGCCCAC
CCGCAAGCATTACCAGCCCTATGCCCCACCACGCGACTTCGCAGCCTATCGCTCC(SEQ ID
NO: 24)-
hCD3 zeta: 
CTGAGAGTGAAGTTCAGCAGGAGCGCAGACGCCCCCGCGTACCAGCAGGGCCAGAACCAGCTCTA
TAACGAGCTCAATCTAGGACGAAGAGAGGAGTACGATGTTTTGGACAAGAGACGTGGCCGGGACC
CTGAGATGGGGGGAAAGCCGCAGAGAAGGAAGAACCCTCAGGAAGGCCTGTACAATGAACTGCAG
AAAGATAAGATGGCGGAGGCCTACAGTGAGATTGGGATGAAAGGCGAGCGCCGGAGGGGCAAGGG
GCACGATGGCCTTTACCAGGGTCTCAGTACAGCCACCAAGGACACCTACGACGCCCTTCACATGC
AGGCCCTGCCCCCTCGC (SEQ ID NO: 25)TAA-
XhoI site: TCGATTCTCGAG (SEQ ID NO: 26)

### 28z CAR Amino Acid Sequence

mVH leader: MNFGLRLIFLVLTLKGVKC (SEQ ID NO: 27)- mVH: 
EVQLVESGPGLVAPSQSLSITCTVSGFSLPSYGVHWVRQPPGKGLEWLGVIWAGGITNYNSALMS
RLTISKDNSKSQVFLKMNSLQTDDTAIYYCARGGSDYDGFAYWGQGTLVTVS (SEQ ID NO:
28)-
single chain: GGGGSGGGGSGGGGS (SEQ ID NO: 29)- mVkappa: 
DIVMTQSPSSLAVSAGEKVTMNCRSSQSLLSSRTRKNYLAWYQQKPGQSPKLLIYWASIRESGVP
DRFTGSGSGTDFTLTISSVQAEDLAVYYCKQSYNLRTFGGGTKLEIK (SEQ ID NO: 30)-
hCkappa: 
RTVAAPSVFIFPPSDEQLKSGTASVVCLLNNFYPREAKVQWKVDNALQSGNSQESVTEQDSKDST
YSLSSTLTLSKADYEKHKLYACEVTHQGLSSPVTKSFNRGECGAP (SEQ ID NO: 31)-
hCD28 transmembrane: TRFWVLVVVGGVLACYSLLVTVAFIIFWVR (SEQ ID NO: 32)-
hCD28 intracellular domain:
   SKRSRLLHSDYMNMTPRRPGPTRKHYQPYAPPRDFAAYRS (SEQ ID NO: 33)- hCD3 zeta:

LRVKFSRSADAPAYQQGQNQLYNELNLGRREEYDVLDKRRGRDPEMGGKPQRRKNPQEGLYNELQ
KDKMAEAYSEIGMKGERRRGKGHDGLYQGLSTATKDTYDALHMQALPPR* (SEQ ID NO:
34)-

### Artificial Gene Base Sequence for Preparing zG CAR

NotI site kozak sequence: GCGGCCGCCACC (SEQ ID NO: 17)- mVH leader:
ATGAACTTTGGGCTCAGATTGATTTTCCTTGTCCTTACTTTAAAAGGTGTGAAGTGT (SEQ ID NO: 18)-
mVH: 
GAGGTGCAGCTGGTGGAGTCTGGACCTGGCCTGGTGGCGCCCTCACAGAGCCTGTCCATCACTTG
CACTGTCTCTGGGTTTTCATTACCCAGCTATGGTGTTCACTGGGTTCGCCAGCCTCCAGGAAAGG
GTCTGGAGTGGCTGGGAGTAATCTGGGCTGGTGGAATCACAAATTATAACTCGGCTCTCATGTCC
AGACTGACCATCAGCAAAGACAACTCCAAGAGCCAAGTTTTCTTAAAAATGAACAGTCTTCAAAC
TGATGACACAGCCATATACTACTGTGCCAGAGGCGGCTCTGATTACGACGGCTTTGCTTACTGGG
GCCAAGGGACTCTGGTCACTGTCTCTGCATCA (SEQ ID NO: 19)-
single chain: GGAGGTGGAGGTTCTGGTGGAGGAGGTTCAGGTGGAGGTGGATCA (SEQ ID NO: 20)-
mVkappa: 
GACATTGTGATGACACAGTCTCCATCCTCCCTGGCTGTGTCAGCAGGAGAGAAGGTCACTATGAA
CTGCAGATCCAGTCAGAGTCTCCTCAGCAGTAGAACCCGAAAGAACTACTTGGCTTGGTACCAGC
AGAAACCAGGGCAGTCTCCTAAACTGCTGATCTACTGGGCATCTATTAGGGAATCTGGGGTCCCT
GATCGCTTCACAGGCAGTGGATCTGGGACAGATTTCACTCTCACCATCAGCAGTGTGCAGGCTGA
AGACCTGGCAGTTTATTACTGCAAGCAATCT TAT AAT CTT CGG ACG TTC GGT GGA
GGC ACC AAG CTG GAA ATC AAA (SEQ ID NO: 21)-
hCkappa: 
CGAACTGTGGCTGCACCATCTGTCTTCATCTTCCCGCCATCTGATGAGCAGTTGAAATCTGGAAC
TGCCTCTGTTGTGTGCCTGCTGAATAACTTCTATCCCAGAGAGGCCAAAGTACAGTGGAAGGTGG
ATAACGCCCTCCAATCGGGTAACTCCCAGGAGAGTGTCACAGAGCAGGACAGCAAGGACAGCACC
TACAGCCTCAGCAGCACCCTGACGCTGAGCAAAGCGGACTACGAGAAACACAAACTCTACGCCTG
CGAAGTCACCCATCAGGGCCTGAGCTCGCCCGTCACAAAGAGCTTCAACAGGGGAGAGTGTGGCG
CGCCA (SEQ ID NO: 22)-
hCD28 transmembrane: 
ACTAGATTTTGGGTGCTGGTGGTGGTTGGTGGAGTCCTGGCTTGCTATAGCTTGCTAGTAACAGT
GGCCTTTATTATTTTCTGGGTGAGG (SEQ ID NO: 23)-
hCD3 zeta: 
CTGAGAGTGAAGTTCAGCAGGAGCGCAGACGCCCCCGCGTACCAGCAGGGCCAGAACCAGCTCTA
TAACGAGCTCAATCTAGGACGAAGAGAGGAGTACGATGTTTTGGACAAGAGACGTGGCCGGGACC
CTGAGATGGGGGGAAAGCCGCAGAGAAGGAAGAACCCTCAGGAAGGCCTGTACAATGAACTGCAG
 
AAAGATAAGATGGCGGAGGCCTACAGTGAGATTGGGATGAAAGGCGAGCGCCGGAGGGGCAAGGG
GCACGATGGCCTTTACCAGGGTCTCAGTACAGCCACCAAGGACACCTACGACGCCCTTCACATGC
AGGCCCTGCCCCCTCGC (SEQ ID NO: 25)-
hGITR intracellular domain: 
AGGAGTCAGTGCATGTGGCCCCGAGAGACCCAGCTGCTGCTGGAGGTGCCGCCGTCGACCGAAGA
CGCCAGAAGCTGCCAGTTCCCCGAGGAAGAGCGGGGCGAGCGATCGGCAGAGGAGAAGGGGCGGC
TGGGAGACCTGTGGGTG (SEQ ID NO: 35)TAA-
XhoI site: TCGATTCTCGAG (SEQ ID NO: 26)

### zG CAR Amino Acid Sequence

mVH leader: MNFGLRLIFLVLTLKGVKC (SEQ ID NO: 27)- mVH: 
EVQLVESGPGLVAPSQSLSITCTVSGFSLPSYGVHWVRQPPGKGLEWLGVIWAGGITNYNSALMS
RLTISKDNSKSQVFLKMNSLQTDDTAIYYCARGGSDYDGFAYWGQGTLVTVS (SEQ ID NO:
28)-
single chain: GGGGSGGGGSGGGGS (SEQ ID NO: 29)-mVkappa: 
DIVMTQSPSSLAVSAGEKVTMNCRSSQSLLSSRTRKNYLAWYQQKPGQSPKLLIYWASIRESGVP
DRFTGSGSGTDFTLTISSVQAEDLAVYYCKQSYNLRTFGGGTKLEIK (SEQ ID NO: 30)-
hCkappa: 
RTVAAPSVFIFPPSDEQLKSGTASVVCLLNNFYPREAKVQWKVDNALQSGNSQESVTEQDSKDST
YSLSSTLTLSKADYEKHKLYACEVTHQGLSSPVTKSFNRGECGAP (SEQ ID NO: 31)-
hCD28 transmembrane: TRFWVLVVVGGVLACYSLLVTVAFIIFWVR (SEQ ID NO: 32)-
hCD3 zeta: 
LRVKFSRSADAPAYQQGQNQLYNELNLGRREEYDVLDKRRGRDPEMGGKPQRRKNPQEGLYNELQ
KDKMAEAYSEIGMKGERRRGKGHDGLYQGLSTATKDTYDALHMQALPPR (SEQ ID NO:
34)-
hGITR intracellular domain:
   RSQCMWPRETQLLLEVPPSTEDARSCQFPEEERGERSAEEKGRLGDLWV* (SEQ ID NO: 36)

For 28z GITRL CAR, an expression plasmid in which the following P2A-GITRL base sequence was incorporated adjacent to the 3' side of the base sequence of SEQ ID NO: 25 of the 28z CAR expression plasmid was prepared using the artificial gene and PCR.
P2A-GITRL base sequence:

GGATCCGGCGCCACAAATTTTAGCCTCTTGAAGCAAGCCGGCGACGTGGAAGAGAATCCTGGCCC
C (P2A base sequence: SEQ ID NO: 37)-

ATGACCCTGCACCCCAGCCCCATCACCTGCGAGTTCCTGTTCAGCACCGCCCTGATCAGCCCCAA
GATGTGCCTGAGCCACCTGGAGAACATGCCCCTGAGCCACAGCAGAACCCAGGGCGCCCAGAGAA
GCAGCTGGAAGCTGTGGCTGTTCTGCAGCATCGTGATGCTGCTGTTCCTGTGCAGCTTCAGCTGG
CTGATCTTCATCTTCCTGCAGCTGGAGACCGCCAAGGAGCCCTGCATGGCCAAGTTCGGCCCCCT
GCCCAGCAAGTGGCAGATGGCCAGCAGCGAGCCCCCCTGCGTGAACAAGGTGAGCGACTGGAAGC
TGGAGATCCTGCAGAACGGCCTGTACCTGATCTACGGCCAGGTGGCCCCCAACGCCAACTACAAC
GACGTGGCCCCCTTCGAGGTGAGACTGTACAAGAACAAGGACATGATCCAGACCCTGACCAACAA
GAGCAAGATCCAGAACGTGGGCGGCACCTACGAGCTGCACGTGGGCGACACCATCGACCTGATCT
TCAACAGCGAGCACCAGGTGCTGAAGAACAACACCTACTGGGGCATCATCCTGCTGGCCAACCCC
CAGTTCATCAGC (GITRL base sequence: SEQ ID NO: 38)

The amino acid sequence of P2A-GITRL is as follows: GSGATNFSLLKQAGDVEENPGP (P2A amino acid sequence: SEQ ID NO: 39)-MTLHPSPITCEFLFSTALISPKMCLSHLENMPLSHSRTQGAQRSSWKLWLFCSIVMLLFLCSFSW LIFIFLQLETAKEPCMAKFGPLPSKWQMASSEPPCVNKVSDWKLEILQNGLYLIYGQVAPNANYN DVAPFEVRLYKNKDMIQTLTNKSKIQNVGGTYELHVGDTIDLIFNSEHQVLKNNTYWGIILLANP QFIS (GITRL amino acid sequence: SEQ ID NO: 40).

For zG GITRL CAR, an expression plasmid in which the P2A-GITRL base sequence was incorporated adjacent to the 3' side of the base sequence of SEQ ID NO: 35 of the zG CAR expression plasmid was prepared using the artificial gene and PCR.

### Test Example 8: Introduction of CAR Gene into T Cell and Confirmation of Expression

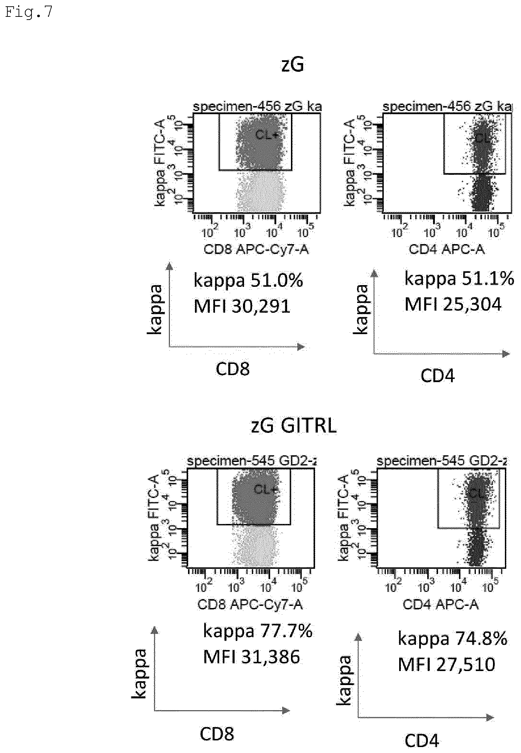

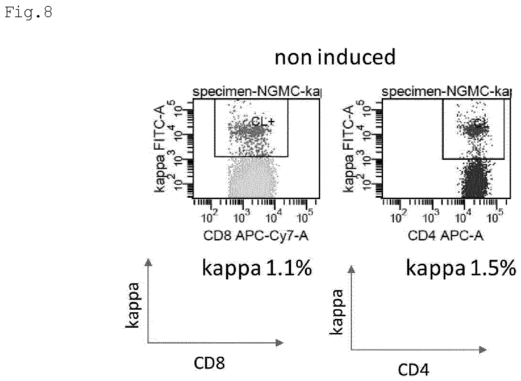

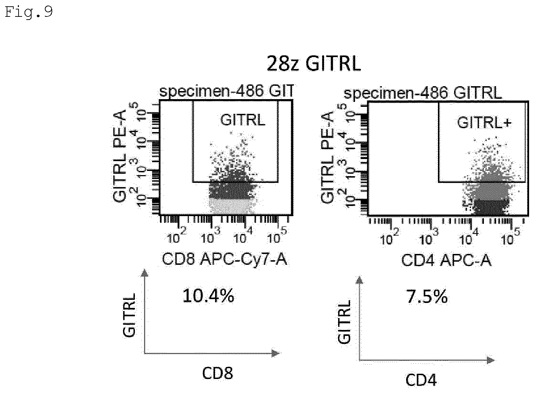

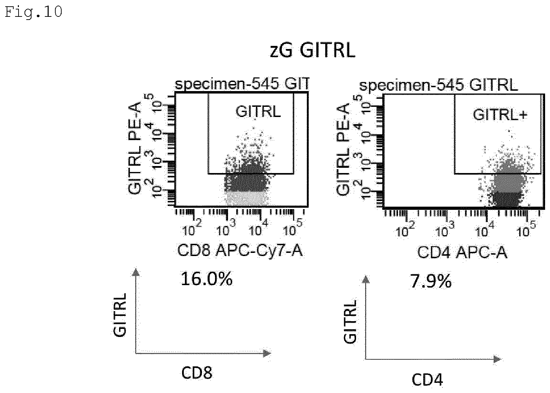

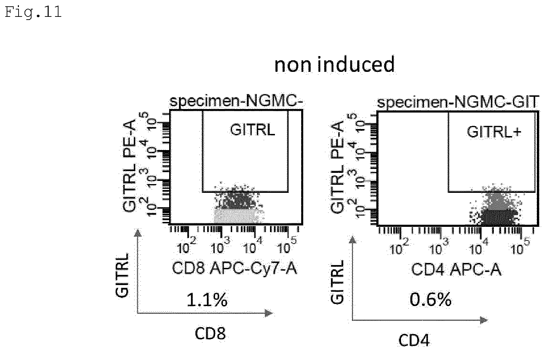

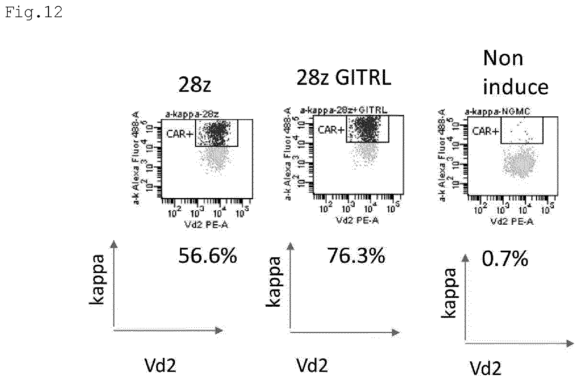

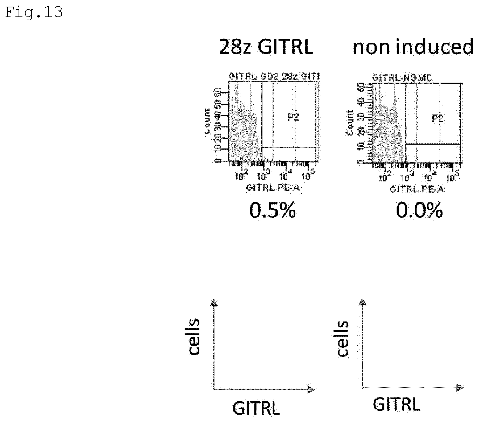

Each plasmid DNA constructed as mentioned above was introduced into Plat-A cells to prepare retroviruses. Cultured human PBMCs were infected with the retroviruses to obtain CAR-transduced T cells, and CAR expression was examined by flow cytometry. CAR and ligand expression was confirmed in alpha/beta T cells as shown in Figs. 6 to 11, and in gamma/delta T cells as shown in Figs. 12 and 13. These are effector cells. It was confirmed that the expression efficiency and expression intensity (indicated by mean fluorescent intensity; MFI) of the CARs were enhanced by co-expression with GITRL (Figs. 6 to 8).

### Test Example 9. Recognition of Target Cell by Effector Cell

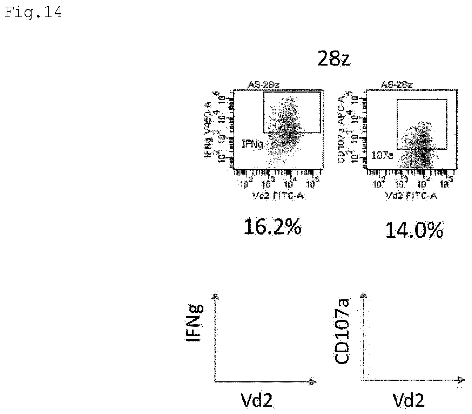

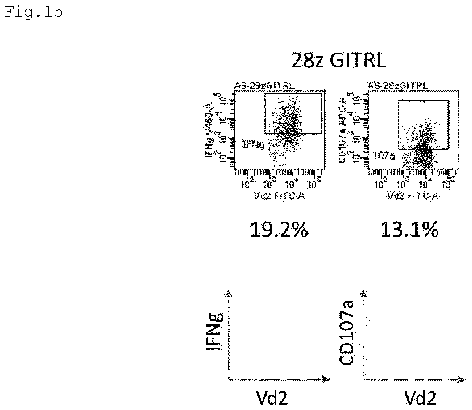

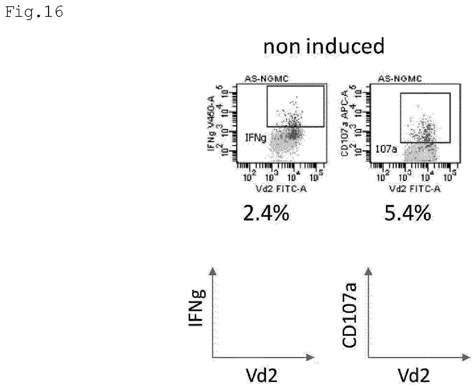

CAR-T cells are activated and express IFNg and TNFa when co-cultured with target AS cells. In addition, CD107a is transported to the cell surface. These reactions indicate that multifunctional reactions have occurred. It was confirmed that all kinds of the CAR-T cells produced in this experiment were activated by co-culture with AS cells, and that these reactions occurred (Figs. 14 to 16).

### Test Example 10: Confirmation of Cytotoxic Action of Effector Cell

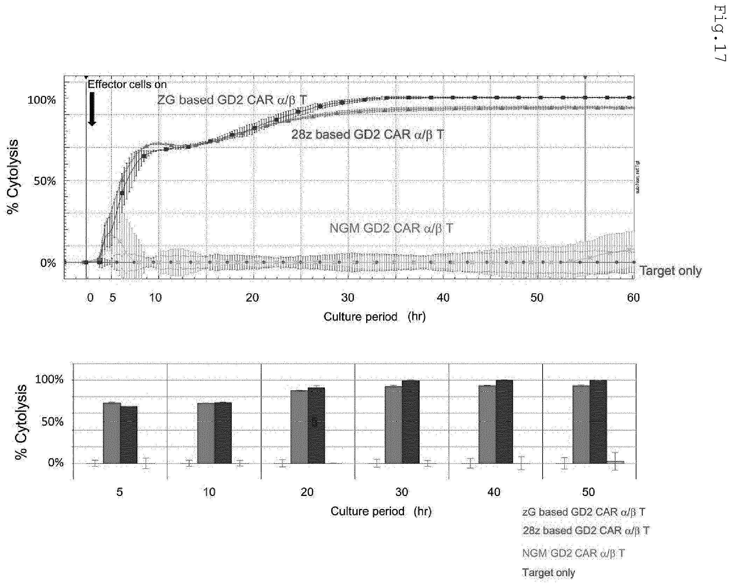

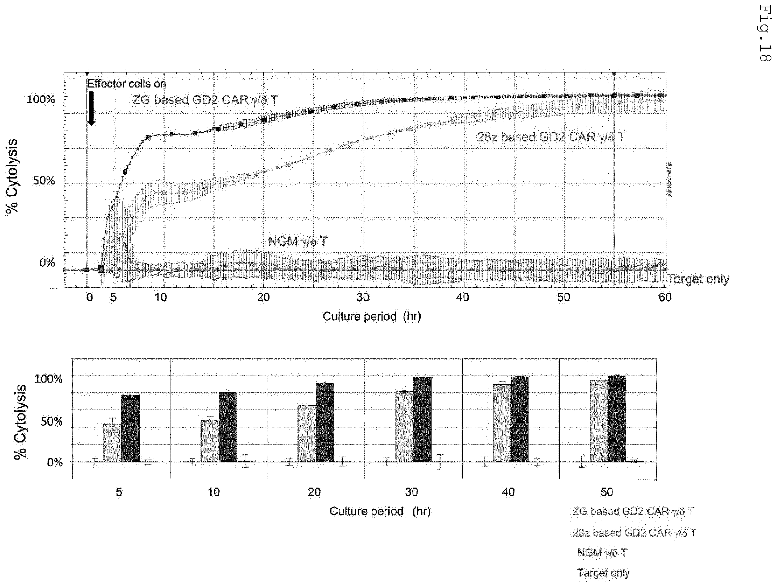

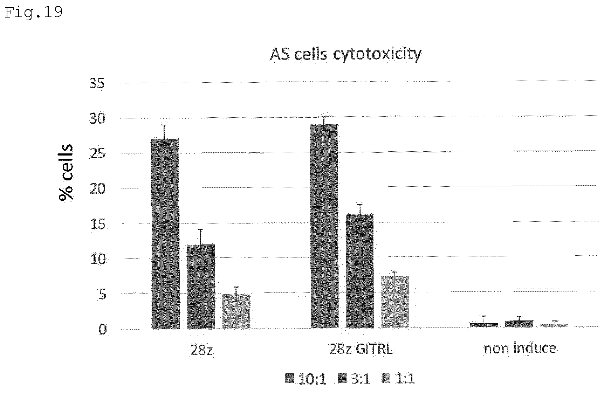

The cytotoxic action of the effector cells on AS cells was examined according to changes over time by using xCELLigence. The results showed that GD2 CAR had sufficient cytotoxic activity in alpha/beta T cells and gamma/delta T cells (Figs. 17 and 18). This was also observed in a non-radioactive cytotoxicity test (Fig. 19).

### Test Example 11. Analysis 1 of Cytotoxic Action of Effector Cell

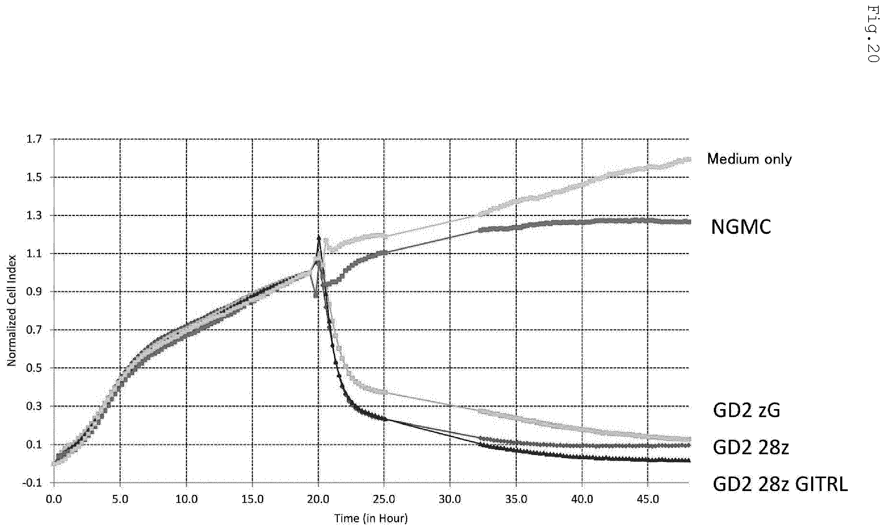

After GD2-positive Kelly cells (20000 cells) were cultured on an E-plate for 20 hours, each kind of the effector cells (alpha/beta) (40000 cells) was added and cultured, and the cell index was tracked over time. The cell index reflects the number of Kelly cells on the E-plate. The normalized cell index is a cell index normalized on the assumption that the number of Kelly cells immediately before co-culture with the effector cells was 1. The graph shows the average values (n = 2). Effective cytotoxicity by GD2 28z, GD2 zG, and GITRL-co-expressing GD2 28z CAR-T cells was observed, and no cytotoxicity by PBMCs into which a CAR was not introduced was observed (Fig. 20).

### Test Example 12: Analysis 2 of Cytotoxic Action of Effector Cell

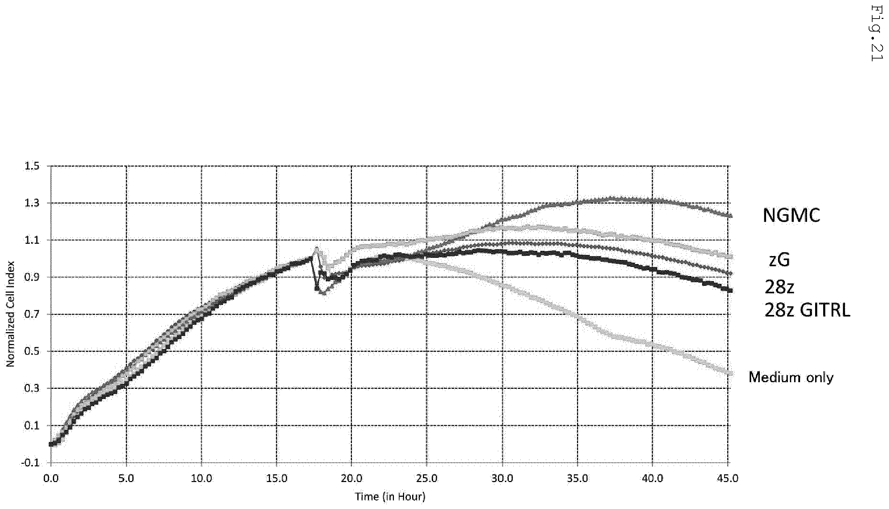

After GD2-negative SK-N-SH cells (20000 cells) were cultured on an E-plate for 18 hours, each kind of the effector cells (alpha/beta) (40000 cells) was allowed to act thereon, and the cell index was tracked over time. The cell index reflects the number of SK-N-SH cells on the E-plate. The normalized cell index is a cell index normalized on the assumption that the number of SK-N-SH cells immediately before co-culture with the effector cells was 1. The graph shows the average values (n = 2). No cytotoxicity by GD2 28z, GD2 zG, or GITRL-co-expressing GD2 28z CAR-T cells was observed (Fig. 21).

### Test Example 13: Analysis 3 of Cytotoxic Action of Effector Cell

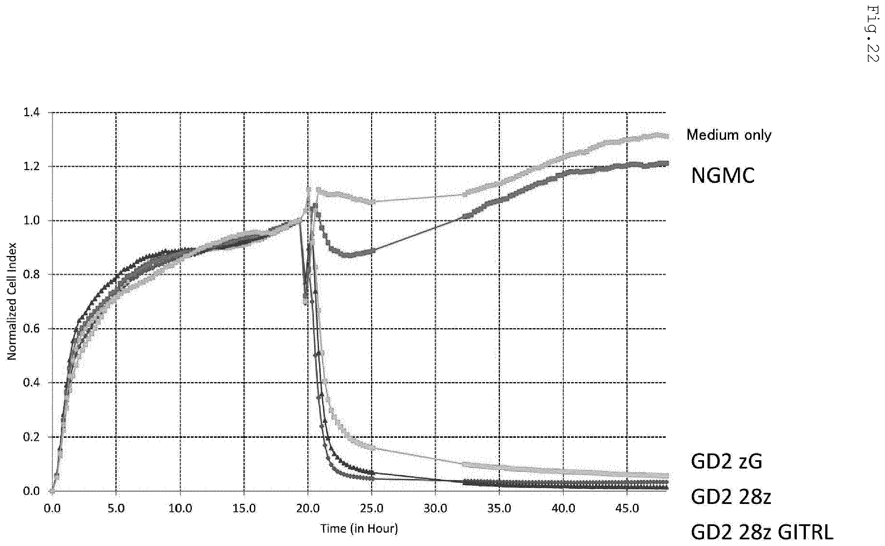

After GD2-positive Hs578T-Luc cells (15000 cells) were cultured on an E-plate for 20 hours, each kind of the effector cells (alpha/beta) (40000 cells) was allowed to act thereon, and the cell index was tracked over time. The cell index reflects the number of Hs578T-Luc cells on the E-plate. The normalized cell index is a cell index normalized on the assumption that the number of Hs578T-Luc cells immediately before co-culture with the effector cells was 1. The graph shows the average values (n = 2). Effective cytotoxicity by GD2 28z, GD2 zG, and GITRL-co-expressing GD2 28z CAR-T cells was observed, and no cytotoxicity by PBMCs into which a CAR was not introduced was observed (Fig. 22).

### Test Example 14: Analysis 4 of Cytotoxic Action of Effector Cell

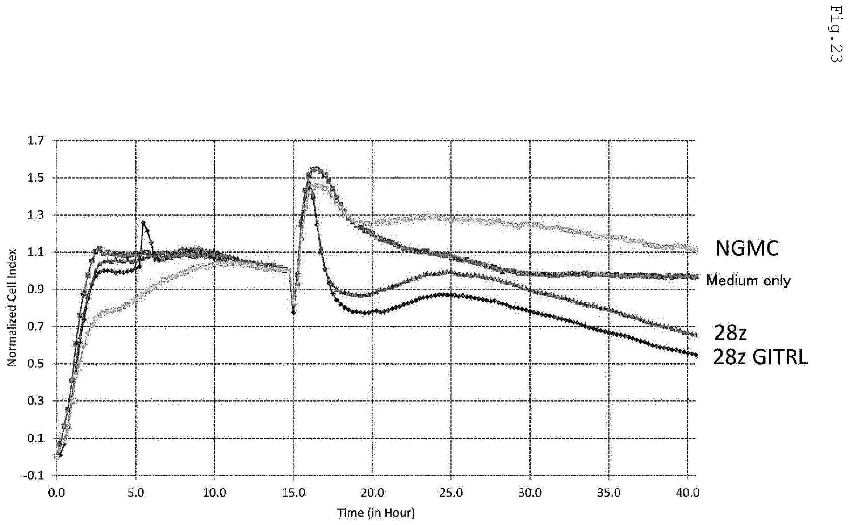

After GD2-negative BT549-Luc cells (20000 cells) were cultured on an E-plate for 18 hours, each kind of the effector cells (alpha/beta) (40000 cells) was allowed to act thereon, and the cell index was tracked over time. The cell index reflects the number of BT549-Luc cells on the E-plate. The normalized cell index is a cell index normalized on the assumption that the number of BT549-Luc cells immediately before co-culture with the effector cells was 1. The graph shows the average values (n = 2). No cytotoxicity by GD2 28z, or GITRL-co-expressing GD2 28z CAR-T cells was observed (Fig. 23).

### Test Example 15: Analysis 5 of Cytotoxic Action of Effector Cell

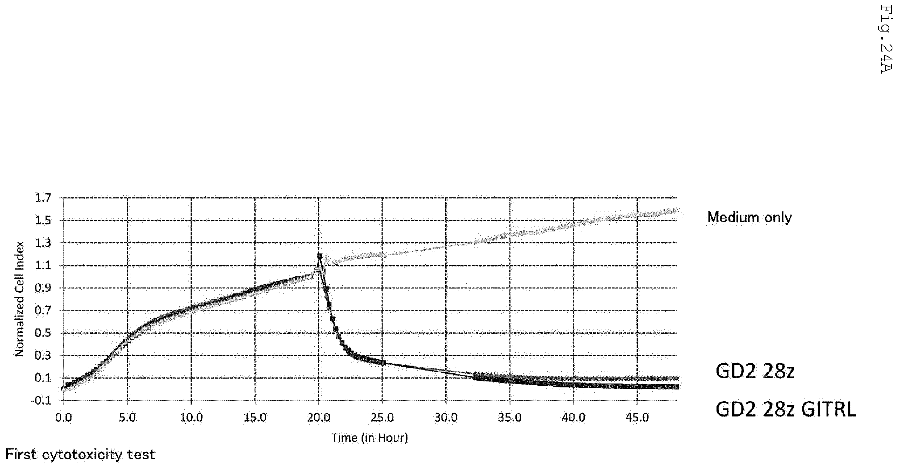

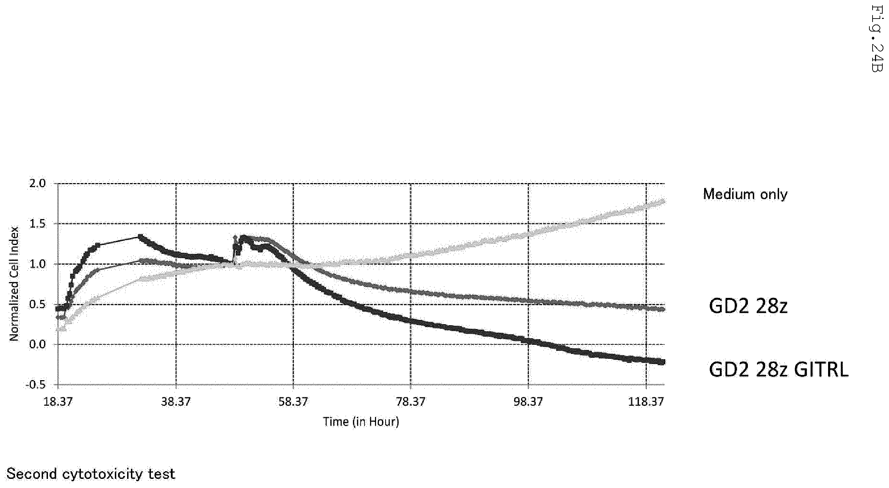

After GD2-positive Kelly cells (20000 cells) were cultured on an E-plate for 20 hours, each kind of the effector cells (alpha/beta) (30000 cells) was allowed to act thereon, and the cell index was tracked over time. One day later, effective cytotoxicity by GD2 28z and GITRL-co-expressing GD2 28z CAR-T cells was observed. The effector cells were collected and successively co-cultured with Kelly cells cultured on an E-plate for 24 hours, and changes in the cell index were recorded over time. The cell index reflects the number of Kelly cells on the E-plate. The normalized cell index is a cell index normalized on the assumption that the number of Kelly cells immediately before co-culture with the effector cells was 1. The graph shows the average values (n = 2). In the second successive cytotoxicity test, the GITRL-co-expressing 28z CAR-T cells retained stronger cytotoxic activity than 28z CAR (Fig. 24).

### Test Example 16: Analysis 6 of Cytotoxic Action of Effector Cell

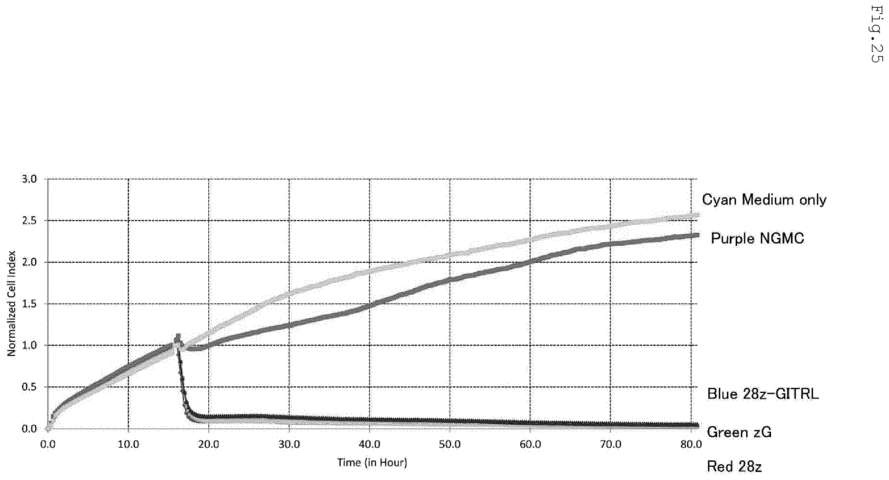

D8 cells are a GD2-positive cell line established by introduction of GD3 synthase and GD2 synthase genes into GD2-negative small-cell lung cancer SK-LC-17, and G418 selection. After GD2-positive D8 cells (10000 cells) were cultured on an E-plate for 18 hours, each kind of the effector cells (alpha/beta) (30000 cells) was allowed to act thereon, and the cell index was tracked over time. The cell index reflects the number of D8 cells on the E-plate. The normalized cell index is a cell index normalized on the assumption that the number of D8 cells immediately before co-culture with the effector cells was 1. The graph shows the average values (n = 2). Effective cytotoxicity by GD2 28z, GD2 zG, and GITRL-co-expressing GD2 28z CAR-T cells was observed, and no cytotoxicity by PBMCs into which a CAR was not introduced was observed (Fig. 25).

### Test Example 17: Analysis 6 of Cytotoxic Action of Effector Cell

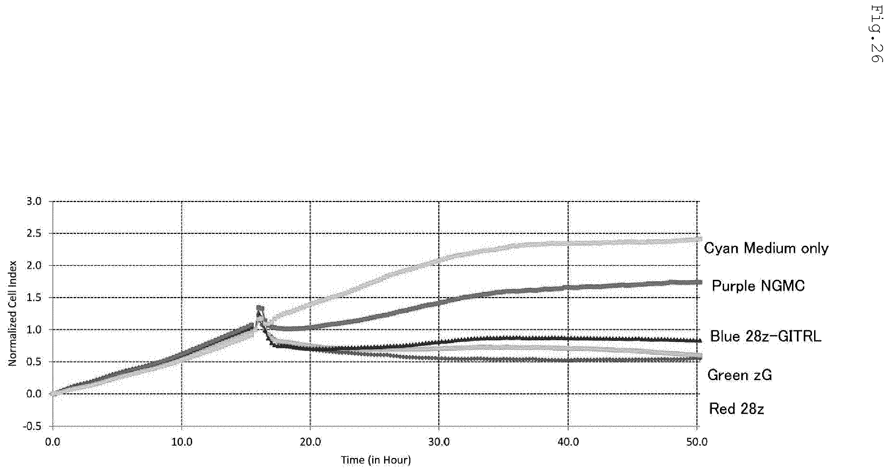

C2 cells are a GD2-negative cell line established by introduction of a pCDNA3.1neo plasmid into GD2-negative small-cell lung cancer SK-LC-17, and G418 selection. After GD2-negative C2 cells (10000 cells) were cultured on an E-plate for 18 hours, each kind of the effector cells (alpha/beta) (30000 cells) was allowed to act thereon, and the cell index was tracked over time. The cell index reflects the number of C2 cells on the E-plate. The normalized cell index is a cell index normalized on the assumption that the number of C2 cells immediately before co-culture with the effector cells was 1. The graph shows the average values (n = 2). No cytotoxicity by GD2 28z, GD2 zG, GITRL-co-expressing GD2 28z CAR-T cells, or PBMCs into which a CAR was not introduced was observed (Fig. 26).

### Test Example 18: Analysis 7 of Cytotoxic Action of Effector Cell

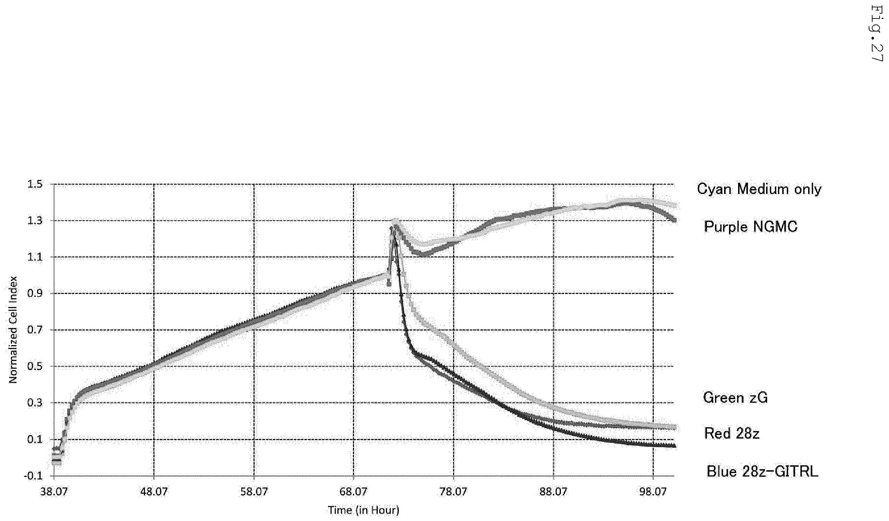

After GD2-positive NCI-N417 cells (20000 cells) were cultured on an E-plate for 34 hours, each kind of the effector cells (alpha/beta) (60000 cells) was allowed to act thereon, and the cell index was tracked over time. The cell index reflects the number of NCI-N417 cells on the E-plate. The normalized cell index is a cell index normalized on the assumption that the number of NCI-N417 cells immediately before co-culture with the effector cells was 1. The graph shows the average values (n = 2). Effective cytotoxicity by GD2 28z, GD2 zG, and GITRL-co-expressing GD2 28z CAR-T cells was observed, and no cytotoxicity by PBMCs into which a CAR was not introduced was observed (Fig. 27).

## Claims

1. A chimeric antigen receptor comprising a GD2-binding domain comprising
a heavy-chain variable region containing
a heavy-chain CDR1 containing the amino acid sequence represented by SEQ ID NO: 1,
a heavy-chain CDR2 containing the amino acid sequence represented by SEQ ID NO: 2, and
a heavy-chain CDR3 containing the amino acid sequence represented by SEQ ID NO: 3, and
a light-chain variable region containing
a light-chain CDR1 containing the amino acid sequence represented by SEQ ID NO: 9,
a light-chain CDR2 containing the amino acid sequence represented by SEQ ID NO: 10, and
a light-chain CDR3 containing the amino acid sequence represented by SEQ ID NO: 11.

2. The chimeric antigen receptor according to claim 1, wherein the binding capability of the chimeric antigen receptor to ganglioside GD1a, ganglioside GD1b, ganglioside GD3, ganglioside GM1, ganglioside GM3, ganglioside GT1b, or lactosylceramide is equal to or less than 1/2 of the binding capability of the chimeric antigen receptor to ganglioside GD2.

3. The chimeric antigen receptor according to claim 1 or 2, comprising a core domain containing
a scFv domain that contains the heavy-chain variable region and the light-chain variable region,
a transmembrane domain, and
an intracellular domain of TCR.

4. The chimeric antigen receptor according to claim 3, wherein the core domain further contains an intracellular domain of a co-stimulator.

5. The chimeric antigen receptor according to claim 3 or 4, comprising a GITRL domain at the C-terminus side of the core domain via a self-cleaving peptide domain.

6. A polynucleotide encoding the chimeric antigen receptor of any one of claims 1 to 5.

7. A cell comprising the polynucleotide of claim 6.

8. A chimeric antigen receptor T-cell or chimeric antigen receptor NK-cell comprising the polynucleotide of claim 6.

9. A pharmaceutical composition comprising the chimeric antigen receptor T-cell or the chimeric antigen receptor NK-cell of claim 8.

10. The pharmaceutical composition according to claim 9, which is for use in the treatment or prevention of cancer.

## Patentansprüche

1. Chimärer Antigenrezeptor, umfassend eine GD2-Bindungsdomäne, umfassend
eine variable Region der schweren Kette, enthaltend
eine CDR1 der schweren Kette, welche die Aminosäuresequenz enthält, die durch SEQ ID NO: 1 dargestellt ist,
eine CDR2 der schweren Kette, welche die Aminosäuresequenz enthält, die durch SEQ ID NO: 2 dargestellt ist, und
eine CDR3 der schweren Kette, welche die Aminosäuresequenz enthält, die durch SEQ ID NO: 3 dargestellt ist, und
eine variable Region der leichten Kette, enthaltend
eine CDR1 der leichten Kette, welche die Aminosäuresequenz enthält, die durch SEQ ID NO: 9 dargestellt ist,
eine CDR2 der leichten Kette, welche die Aminosäuresequenz enthält, die durch SEQ ID NO: 10 dargestellt ist, und
eine CDR3 der leichten Kette, welche die Aminosäuresequenz enthält, die durch SEQ ID NO: 11 dargestellt ist.

2. Chimärer Antigenrezeptor nach Anspruch 1, wobei die Bindungsfähigkeit des chimären Antigenrezeptors an Gangliosid GD1a, Gangliosid GD1b, Gangliosid GD3, Gangliosid GM1, Gangliosid GM3, Gangliosid GT1b oder Lactosylceramid gleich oder kleiner ist als 1/2 der Bindungsfähigkeit des chimären Antigenrezeptors an Gangliosid GD2.

3. Chimärer Antigenrezeptor nach Anspruch 1 oder 2, die eine Kerndomäne umfasst, enthaltend
eine scFv-Domäne, welche die variable Region der schweren Kette und die variable Region der leichten Kette enthält,
eine Transmembrandomäne, und
eine intrazelluläre Domäne von TCR.

4. Chimärer Antigenrezeptor nach Anspruch 3, wobei die Kerndomäne weiter eine intrazelluläre Domäne eines Co-Stimulators enthält.

5. Chimärer Antigenrezeptor nach Anspruch 3 oder 4, der mittels einer selbstspaltenden Peptiddomäne eine GITRL-Domäne an der C-Terminus-Seite der Kerndomäne umfasst.

6. Polynukleotid, das den chimären Antigenrezeptor nach einem der Ansprüche 1 bis 5 kodiert.

7. Zelle, die das Polynukleotid nach Anspruch 6 umfasst.

8. Chimäre Antigenrezeptor-T-Zelle oder chimäre Antigenrezeptor-NK-Zelle, die das Polynukleotid nach Anspruch 6 umfasst.

9. Pharmazeutische Zusammensetzung, die die chimäre Antigenrezeptor-T-Zelle oder die chimäre Antigenrezeptor-NK-Zelle nach Anspruch 8 umfasst.

10. Pharmazeutische Zusammensetzung nach Anspruch 9, die zur Verwendung bei der Behandlung oder Vorbeugung von Krebs dient.

## Revendications

1. Récepteur antigénique chimérique comprenant un domaine de liaison au GD2 comprenant
une région variable de chaîne lourde contenant
une chaine lourde CDR1 contenant la séquence d'acides aminés représentée par SEQ ID NO : 1,
une chaîne lourde CDR2 contenant la séquence d'acides aminés représentée par SEQ ID NO : 2, et
une chaîne lourde CDR3 contenant la séquence d'acides aminés représentée par SEQ ID NO : 3, et
une région variable de chaîne légère contenant
une chaîne légère CDR1 contenant la séquence d'acides aminés représentée par SEQ ID NO : 9,
une chaîne légère CDR2 contenant la séquence d'acides aminés représentée par SEQ ID NO : 10, et
une chaîne légère CDR3 contenant la séquence d'acides aminés représentée par SEQ ID NO : 11.

2. Récepteur antigénique chimérique selon la revendication 1, dans lequel la capacité de liaison du récepteur antigénique chimérique au ganglioside GD1a, au ganglioside GD1b, au ganglioside GD3, au ganglioside GM1, au ganglioside GM3, au ganglioside GT1b ou au lactosylcéramide est égale ou inférieure à la moitié de la capacité de liaison du récepteur antigénique chimérique au ganglioside GD2.

3. Récepteur antigénique chimérique selon la revendication 1 ou 2, comprenant un domaine central contenant
un domaine scFv qui contient la région variable de chaîne lourde et la région variable de chaîne légère,
un domaine transmembranaire, et
un domaine intracellulaire de TCR.

4. Récepteur antigénique chimérique selon la revendication 3, dans lequel le domaine central contient en outre un domaine intracellulaire d'un co-stimulateur.

5. Récepteur antigénique chimérique selon la revendication 3 ou 4, comprenant un domaine GITRL du côté de l'extrémité C-terminal du domaine central par l'intermédiaire d'un domaine peptidique auto-clivant.

6. Polynucléotide codant pour le récepteur antigénique chimérique de l'une quelconque des revendications 1 à 5.

7. Cellule comprenant le polynucléotide de la revendication 6.

8. Cellule T à récepteur antigénique chimérique ou cellule NK à récepteur antigénique chimérique comprenant le polynucléotide de la revendication 6.

9. Composition pharmaceutique comprenant la cellule T à récepteur antigénique chimérique ou la cellule NK à récepteur antigénique chimérique de la revendication 8.

10. Composition pharmaceutique selon la revendication 9, pour son utilisation pour le traitement ou la prévention du cancer.
